# EUROPEAN PATENT SPECIFICATION

(11) **EP 0 625 505 B1**
(45) Date of publication and mention of the grant of the patent: **28.10.1998**
(21) Application number: 94107449.4
(22) Date of filing: 13.05.1994
(51) Int. Cl.: C07C 323/23, C07C 317/26, A01N 37/34, A01N 41/04, A01N 41/10

(54) **2-Cyano-1,3-dione derivatives and their use as herbicides**
2-Cyan-1,3-dion-Derivate und ihre Verwendung als Herbizid
Dérivés de 2-cyano-1,3-dione et leur emploi en tant qu'herbicides

(30) Priority: 18.05.1993 GB 9310222; 22.07.1993 US 94881
(43) Date of publication of application: 23.11.1994
(60) Divisional application: 97109375.2
(73) Proprietor: RHONE POULENC AGRICULTURE LTD., Ongar, Essex CM5 OHW (GB)
(72) Inventor: Cramp, Susan Mary, Rhône Poulenc, Ongar Essex CM5 0HW (GB); Lambert, Claude, Ibaraki 304 (JP); Little, Gillian Mary, Rhône Poulenc, Ongar Essex CM5 0HW (GB); Morris, John, Rhône Poulenc, Ongar Essex CM5 0HW (GB)
(74) Representative: Brachotte, Charles

(56) References cited:
- EP-A- 0 213 892
- EP-A- 0 496 630
- EP-A- 0 496 631
- BE-A- 819 136
- CHEMICAL ABSTRACTS, vol. 100, no. 20, 14 May 1984, Columbus, Ohio, US; abstract no. 157636t, 'Stabilized chlorine-containing resins' & JP-A-58 201 851 (AKISHIMA KAGAKU KOGYO, K.K.) 24 November 1983
- CHEMICAL ABSTRACTS, vol. 93, no. 18, 3 November 1980, Columbus, Ohio, US; abstract no. 178628u, LOCK, G.A. & THOMPSON, D.W. 'Zirconium complexes with the trifunctional 3-cyanopentane-2,4-dione ligand' page 715 ; & J. CHEM. SOC. DALTON TRANS. vol. 1980, no. 8 , 1980 pages 1265 - 1268
- BULL. CHEM. SOC. JAPAN vol. 44, no. 1 , 1971 pages 185 - 189 SASAKI, T. ET AL. 'Studies on Heteroaromaticity. XLVI. Reactivities of Benzoyl Cyanide N-Oxide and Some Derivatives Therefrom'
- IND. J. CHEM., SECT. B vol. 25B , November 1986 pages 1133 - 1137 SARKAR, M. ET AL. 'Studies on -Enaminonitriles : Part I - Benzoylation in Presence of Sodium in Benzene'
- J. HETEROCYCLIC CHEM. vol. 20, no. 3 , 1983 pages 645 - 648 MENOZZI, G. ET AL. 'Reaction of 2-Dimethylaminomethylene-1,3-diones with Dinucleophiles. II. Synthesis of 5-(Alkyl)(Phenyl)-4-acylisoxazoles and 6,7-Dihydro-1,2-benzisoxazol-4(5H)-ones'

## Description

This invention relates to novel 2-cyano-1,3-dione derivatives, compositions containing them, processes for their preparation and their use as herbicides.

2-Cyano-1,3-diones are disclosed in Chemical Abstracts, Vol. 100 (1984), 157636t; Chemical Abstracts Vol. 93 (1980), 178628u; Bull. Chem. Soc. Jpn., Vol. 44(1) (1971), pp185 - 189; Ind. J. Chem., Vol. 25 (1986), pp1133 - 1137; J Het Chem., Vol. 20 (1983), pp645 - 648; and BE-A-819136. Herbicidal 2-cyano-1,3-diones are described in EP-A-0213892, -0496630 and -0496631.

The present invention provides 2-cyano-1,3-dione derivatives of formula (I): 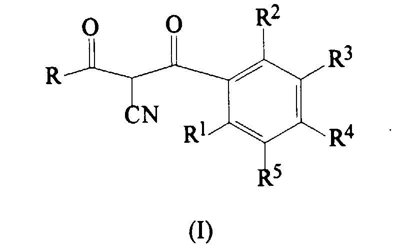 wherein:
R represents:-
   a straight- or branched- chain alkyl group containing up to six carbon atoms which is optionally substituted by one or more halogen atoms; or
   a cycloalkyl group containing from three to six carbon atoms optionally substituted by one or more R⁷ groups;
R¹ represents the hydrogen atom;
R², R³, R⁴ and R⁵, which may be the same or different, each represents:-
   the hydrogen atom;
   a halogen atom;
   a straight- or branched- chain alkyl, alkenyl or alkynyl group containing up to six carbon atoms optionally substituted by one or more halogen atoms;
   phenyl optionally substituted by up to three groups R²¹ which may be the same or different;
   a straight- or branched- chain alkyl group containing up to six carbon atoms which is substituted by a group -OR⁶;
   a group selected from nitro, cyano, -CO₂R⁶,-COR⁷, -S(O)ₙR⁹, -O(CH₂)ₘOR⁶, -N(R¹²)SO₂R⁸, -OR⁶¹, -OSO₂R⁸, -CONR¹⁰R¹⁵ and -(CR¹³R¹⁴)ₜ-S(O)_{q}R⁸;
   provided that at least one of the groups R² to R⁵ represents -(CR¹³R¹⁴)ₜ.S(O)_{q}R⁸;
R⁶ represents a straight- or branched- chain alkyl, alkenyl or alkynyl group containing up to six carbon atoms optionally substituted by one or more halogen atoms; or
   a cycloalkyl group containing from three to six carbon atoms;
R⁶¹ represents:-
   a straight- or branched- chain alkyl, alkenyl or alkynyl group containing up to six carbon atoms optionally substituted by one or more halogen atoms;
   a cycloalkyl group containing from three to six carbon atoms;
   or phenyl optionally substituted by from one to five groups R²¹ which may be the same or different;
R⁷ represents a straight- or branched- chain alkyl group containing up to six carbon atoms optionally substituted by one or more halogen atoms; or
   a cycloalkyl group containing from three to six carbon atoms;
R⁸ represents:-
   a straight- or branched- chain alkyl, alkenyl or alkynyl group containing up to six carbon atoms optionally substituted by one or more halogen atoms;
   a cycloalkyl group containing from three to six carbon atoms;
   phenyl optionally substituted by from one to five groups R²¹ which may be the same or different; or
   -NR¹⁰R¹¹;
R⁹ represents:-
   a straight- or branched- chain alkyl, alkenyl or alkynyl group containing up to six carbon atoms optionally substituted by one or more halogen atoms;
   a cycloalkyl group containing from three to six carbon atoms;
   or phenyl optionally substituted by from one to five groups R²¹ which may be the same or different;
R¹⁰ represents hydrogen a straight- or branched- chain alkyl group containing up to six carbon atoms optionally substituted by one or more halogen atoms;
R¹¹ represents:-
   a straight- or branched- chain alkyl group containing up to six carbon atoms optionally substituted by one or more halogen atoms;
   or a group selected from -COR⁷, -CO₂R⁷ and -CONR⁷R¹⁰;
   where R¹⁰ and R¹¹ are part of a group -NR¹⁰R¹¹ they may, together with the nitrogen to which they are attached, may form a 5 or 6 membered ring optionally having one additional heteroatom in the ring which is oxygen or nitrogen (e.g. pyrrolidine, morpholine, pyrrole, piperidine and piperazine), wherein the ring is optionally substituted by one or more alkyl groups containing up to three carbon atoms;
R¹² represents:
   the hydrogen atom;
   a straight- or branched- chain alkyl, alkenyl or alkynyl group containing up to six carbon atoms optionally substituted by one or more halogen atoms;
   a cycloalkyl group containing from three to six carbon atoms; phenyl optionally substituted by from one to five groups R²¹ which may be the same or different;
   or a group -OR¹⁷;
R¹³ and R¹⁴, which may be the same or different, each represents the hydrogen atom or a straight- or branched- chain alkyl group containing up to six carbon atoms optionally substituted by one or more halogen atoms;
R¹⁵ represents a group selected from R⁷ and -OR¹⁷;
   where R¹⁰ and R¹⁵ are part of a group -CONR¹⁰R¹⁵ they may, together with the nitrogen to which they are attached, form a five or six membered ring optionally having one additional heteroatom in the ring which is oxygen or nitrogen (e.g. pyrrolidine, morpholine, pyrrole, piperidine and piperazine), wherein the ring is optionally substituted by one or more alkyl groups containing up to three carbon atoms;
R¹⁷ represents a straight- or branched- chain alkyl group containing up to six carbon atoms;
R²¹ represents
   a halogen atom;
   a straight- or branched- chain alkyl group containing up to three carbon atoms optionally substituted by one or more halogen atoms;
   or a group selected from nitro, cyano, -S(O)ₙR⁷ and -OR⁷;
m represents one, two or three;
n represents zero, one or two;
q represents zero, one or two; and
t represents an integer from one to four; where t is greater than one the groups -(CR¹³R¹⁴)- may be the same or different.
and agriculturally acceptable salts or metal complexes thereof, which possess valuable properties.

It will be understood that the compounds may exist in enolic tautomeric forms. All such forms are embraced by the present invention.

Compounds of formula I may exist in enolic tautomeric forms that may give rise to geometric isomers around the enolic double bond.

Furthermore in certain cases the groups R¹, R², R³, R⁴ and R⁵ may give rise to stereoisomers and geometric isomers. All such forms are embraced by the present invention.

By the term "agriculturally acceptable salts" is meant salts the cations or anions of which are known and accepted in the art for the formation of salts for agricultural or horticultural use. Preferably the salts are water-soluble. Suitable salts with bases include alkali metal (eg. sodium and potassium), alkaline earth metal (eg. calcium and magnesium), ammonium and amine (eg. diethanolamine, triethanolamine, octylamine, morpholine and dioctylmethylamine) salts. Suitable acid addition salts, formed by compounds of formula (I) containing an amino group, include salts with inorganic acids, for example hydrochlorides, sulphates, phosphates and nitrates and salts with organic acids, for example acetic acid.

By the term "metal complexes" is meant compounds in which one or both of the oxygen atoms of the 1,3-dione act as chelating agents to a metal cation. Examples of such cations include zinc, manganese, cupric, cuprous, ferric, ferrous, titanium and aluminium.

The compounds of the invention, in some aspects of their activity, show advantages over known compounds.

A preferred class of compounds of formula (I) are those wherein:
R represents:-
   a straight- or branched- chain alkyl group containing up to six carbon atoms optionally substituted by one or more halogen atoms;
   or a cycloalkyl group containing from three to six carbon atoms optionally substituted by one or more methyl groups;
R², R³, R⁴ and R⁵, which may be the same or different, each represents:-
   a hydrogen or halogen atom; or
   a straight- or branched- chain alkyl, alkenyl or alkynyl group containing up to six carbon atoms optionally substituted by one or more halogen atoms;
   a straight- or branched- chain alkyl group containing up to six carbon atoms which is substituted by -OR⁶; or
   a group selected from -COR⁷, -CO₂R⁶, cyano, nitro, -O(CH₂)ₘOR⁶; -OR⁶¹, -N(R¹²)SO₂R⁸, -OSO₂R⁸, -S(O)ₙR⁹ and -(CR¹³R¹⁴)ₜSO_{q}R⁸;
R⁶ and R⁷, which may be the same or different each represents:-
   a straight- or branched- chain alkyl group containing up to six carbon atoms optionally substituted by one or more halogen atoms;
   or a cycloalkyl group containing three or four carbon atoms;
R⁶¹ and R⁹, which may be the same or different, each represents:-
   a straight- or branched- chain alkyl or alkenyl group containing up to six carbon atoms optionally substituted by one or more halogen atoms;
   a straight- or branched- alkynyl group containing from three to six carbon atoms optionally substituted by one or more halogen atoms;
   a cycloalkyl group containing three to six carbon atoms;
R⁸ represents:-
   a straight- or branched- chain alkyl or alkenyl group containing up to six carbon atoms optionally substituted by one or more halogen atoms;
   a straight- or branched- alkynyl group containing from three to six carbon atoms optionally substituted by one or more halogen atoms;
   a cycloalkyl group containing three to six carbon atoms; or phenyl optionally substituted by from one to three groups R²¹ which may be the same or different;
R¹² represents:-
   the hydrogen atom;
   a straight- or branched- chain alkyl or alkenyl group containing up to six carbon atoms optionally substituted by one or more halogen atoms;
   a straight- or branched- alkynyl group containing from three to six carbon atoms optionally substituted by one or more halogen atoms;
   a cycloalkyl group containing three to six carbon atoms;
R¹³ and R¹⁴, which may be the same or different, each represents:-
   a hydrogen atom; or
   a straight- or branched- chain alkyl group containing up to six carbon atoms optionally substituted by one or more halogen atoms;
R²¹ represents:-
   a halogen atom;
   a straight- or branched- chain alkyl group containing up to three carbon atoms optionally substituted by one or more halogen atoms; or
   a group selected from nitro, cyano, -S(O)ₙR⁷ and -OR⁷;
m represents two or three;
n represents zero, one or two;
q represents zero, one or two; and
t represents one or two.

A further preferred class of compounds of formula (I) are those wherein:
R represents:-
   a straight- or branched chain alkyl group containing up to three carbon atoms; or
   a cycloalkyl group containing thee or four carbon atoms optionally substituted by one or more methyl groups;
R², R³ and R⁴, which may be the same or different, each represents:-
   a hydrogen, chlorine, bromine or fluorine atom; or
   a straight- or branched- chain alkyl or alkenyl group containing up to four carbon atoms optionally substituted by one or more chlorine, bromine or fluorine atoms;
   a straight- or branched- chain alkynyl group containing up to four carbon atoms;
   a straight- or branched- chain alkyl group containing up to four carbon atoms which is substituted by -OR⁶; or
   a group selected from -COR⁷, -CO₂R⁶, -S(O)ₙR⁹, -O(CH₂)ₘOR⁶, -N(R¹²)SO₂R⁸, -OR⁶¹, -(CR¹³R¹⁴)ₜS(O)_{q}R⁸ and -OSO₂R⁸;
   provided at least one of the groups R² to R⁴ represents -(CR¹³R¹⁴)ₜ-S(O)_{q}R⁸;
R⁵ represents the hydrogen atom;
R⁶, R⁷, R⁸ and R⁹, which may be the same or different, each represents:-
   a straight- or branched- chain alkyl group containing up to four carbon atoms optionally substituted by one or more chlorine, bromine or fluorine atoms; or
   a cyclopropyl group;
R⁶¹ represents:-
   a straight- or branched- chain alkyl or alkenyl group containing up to four carbon atoms optionally substituted by one or more chlorine, bromine or fluorine atoms;
   a straight- or branched- chain alkynyl group containing three or four carbon atoms, or
   a cyclopropyl group;
R¹² represents:-
   the hydrogen atom; or
   a straight- or branched- chain alkyl or alkenyl group containing up to four carbon atoms optionally substituted by one or more chlorine, bromine or fluorine atoms;
   a straight- or branched- chain alkynyl group containing three or four carbon atoms, or
   a cyclopropyl group;
R¹³ and R¹⁴ which may be the same or different, each represents:-
   the hydrogen atom; or
   a straight- or branched- chain alkyl group containing up to three carbon atoms;
m represents two or three;
n represents zero, one or two;
q represents zero, one or two; and
t represents one.

A further preferred class of compounds of formula (I) are those wherein: -
R represents a methyl, ethyl, isopropyl, cyclopropyl or 1-methylcyclopropyl group;
R², R³ and R⁴, which may be the same or different, each represents:-
   a hydrogen, chlorine, bromine or fluorine atom; or
   a straight- or branched- chain alkyl or alkenyl group containing up to four carbon atoms optionally substituted by one or more chlorine, bromine or fluorine atoms;
   a straight- or branched- chain alkyl group containing up to three carbon atoms which is substituted by -OR⁶; or
   a group selected from -COR⁷, -CO₂R⁶, -SR⁹, -O(CH₂)ₘOR⁶, -OR⁶¹, -N(R¹²)SO₂R⁸, -OSO₂R⁸ and -(CR¹³R¹⁴)ₜS(O)_{q}R⁸;
   provided at least one of the groups R² to R⁴ represents -(CR¹³R¹⁴)ₜ-S(O)_{q}R⁸_{;}
R⁵ represents the hydrogen atom;
R⁶, R⁷, R⁸ and R⁹, which may be the same or different, each represents:-
   a straight- or branched- chain alkyl group containing up to three carbon atoms;
R⁶¹ represents:-
   a straight- or branched- chain alkyl group containing up to four carbon atoms optionally substituted by one or more chlorine, bromine or fluorine atoms;
   a straight- or branched- chain alkenyl or alkynyl group containing three or four carbon atoms; or
   a cyclopropyl group;
R¹² represents:-
the hydrogen atom; or
a straight- or branched- chain alkyl group containing up to three carbon atoms optionally substituted by one or more chlorine, bromine or fluorine atoms;
an allyl group optionally substituted by one or more chlorine, bromine or fluorine atoms;
R¹³ and R¹⁴, which may be the same or different, each represents the hydrogen atom, or a methyl or ethyl group;
m represents two or three;
q represents zero, one or two; and
t represents one.

Examples of specific compounds embraced by formula (I) include the following:

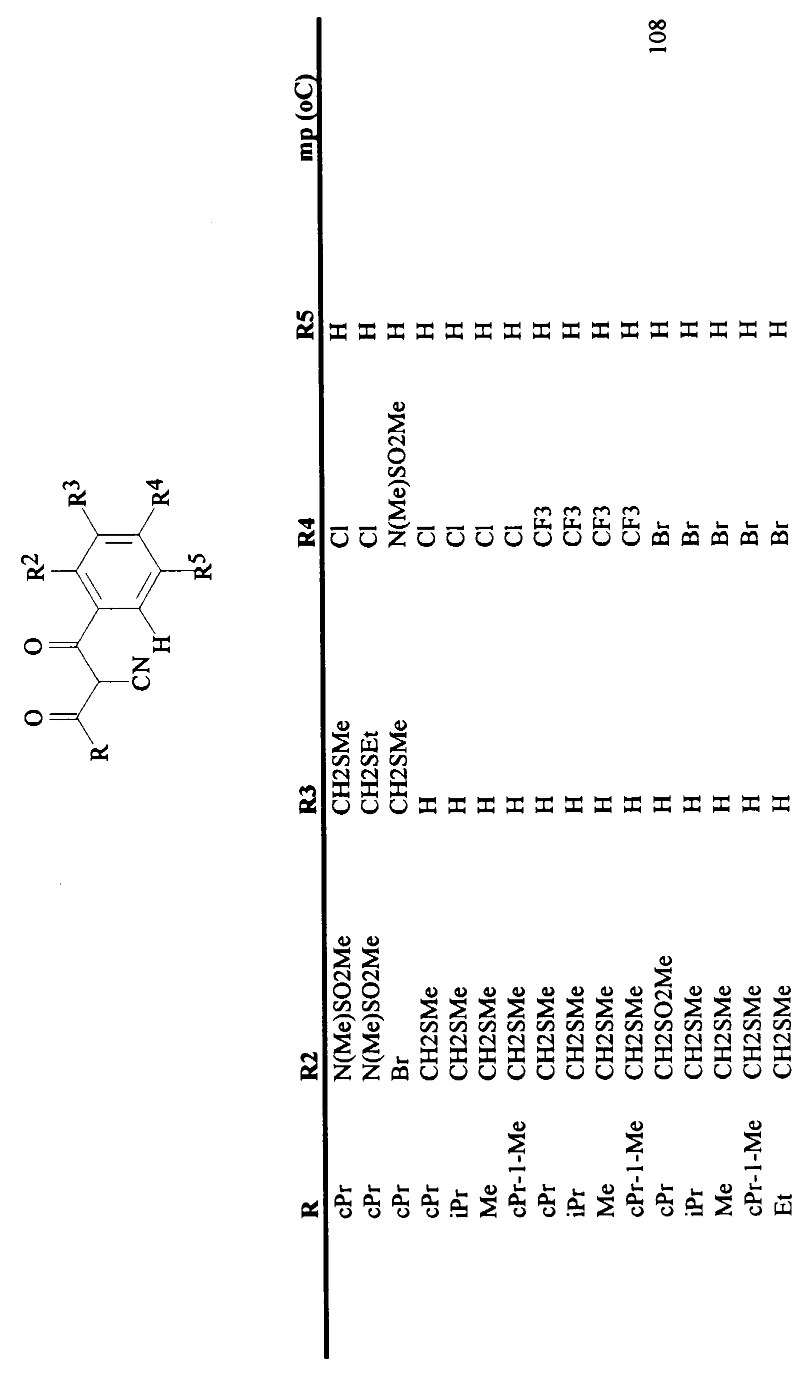

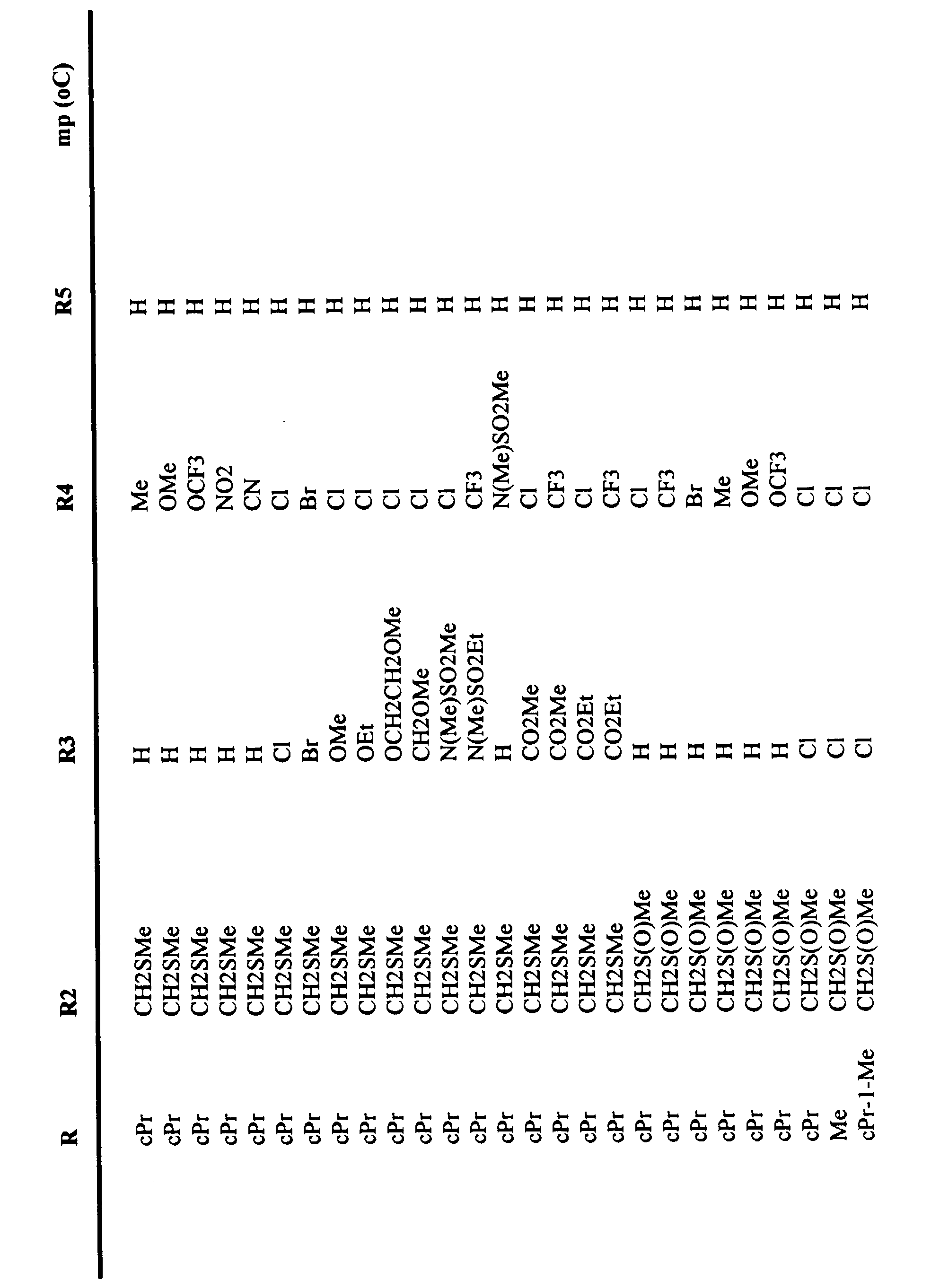

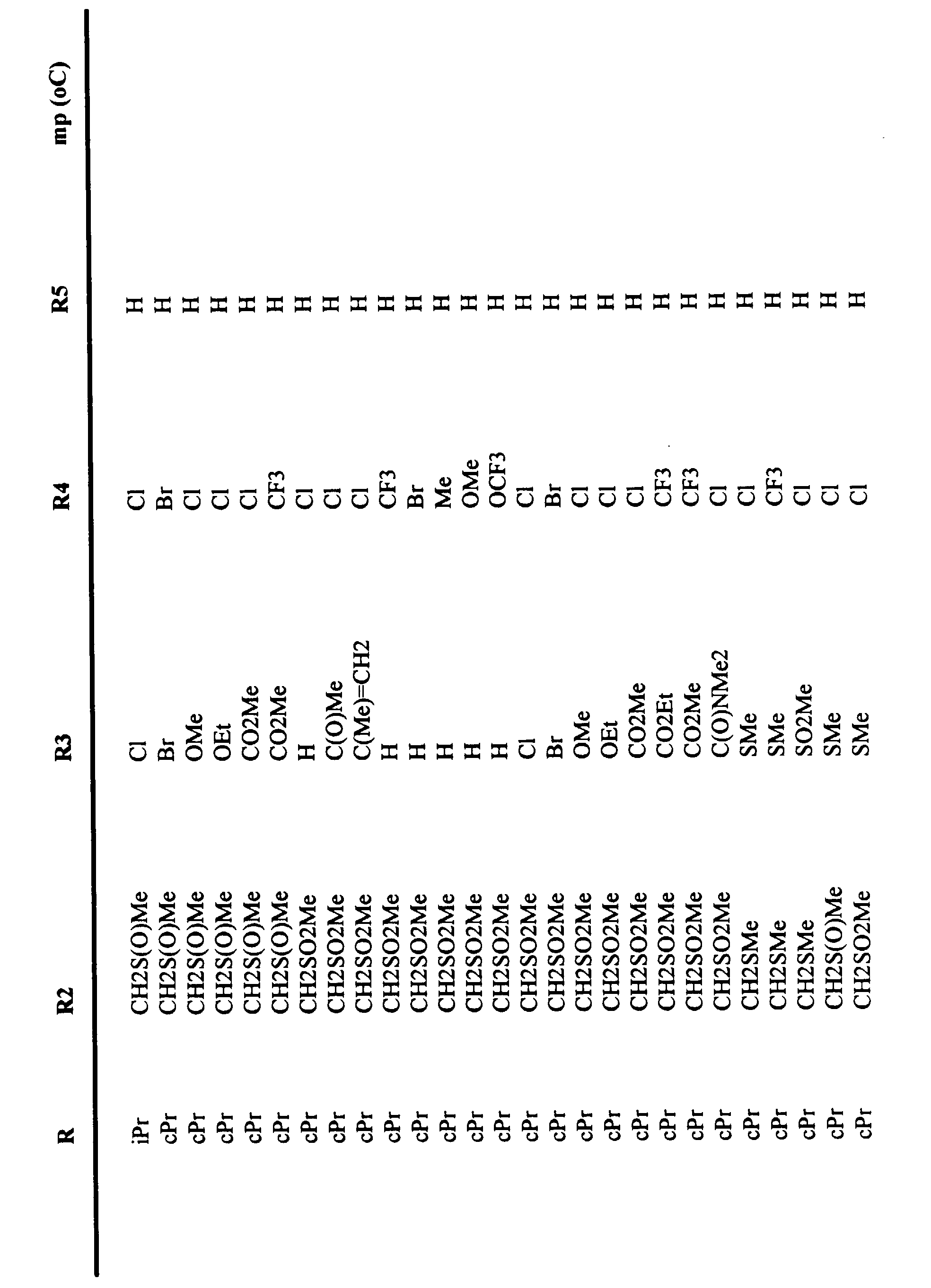

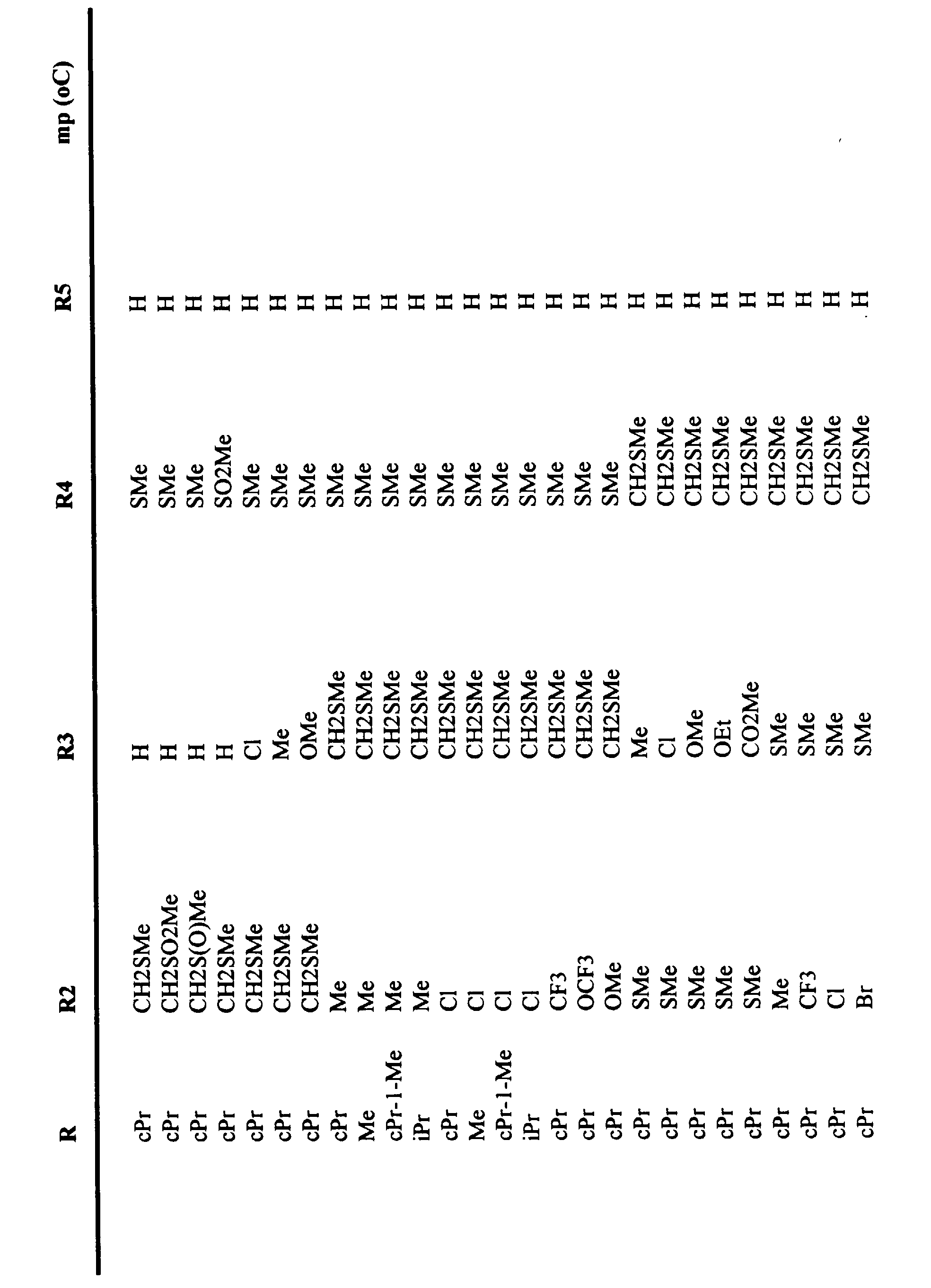

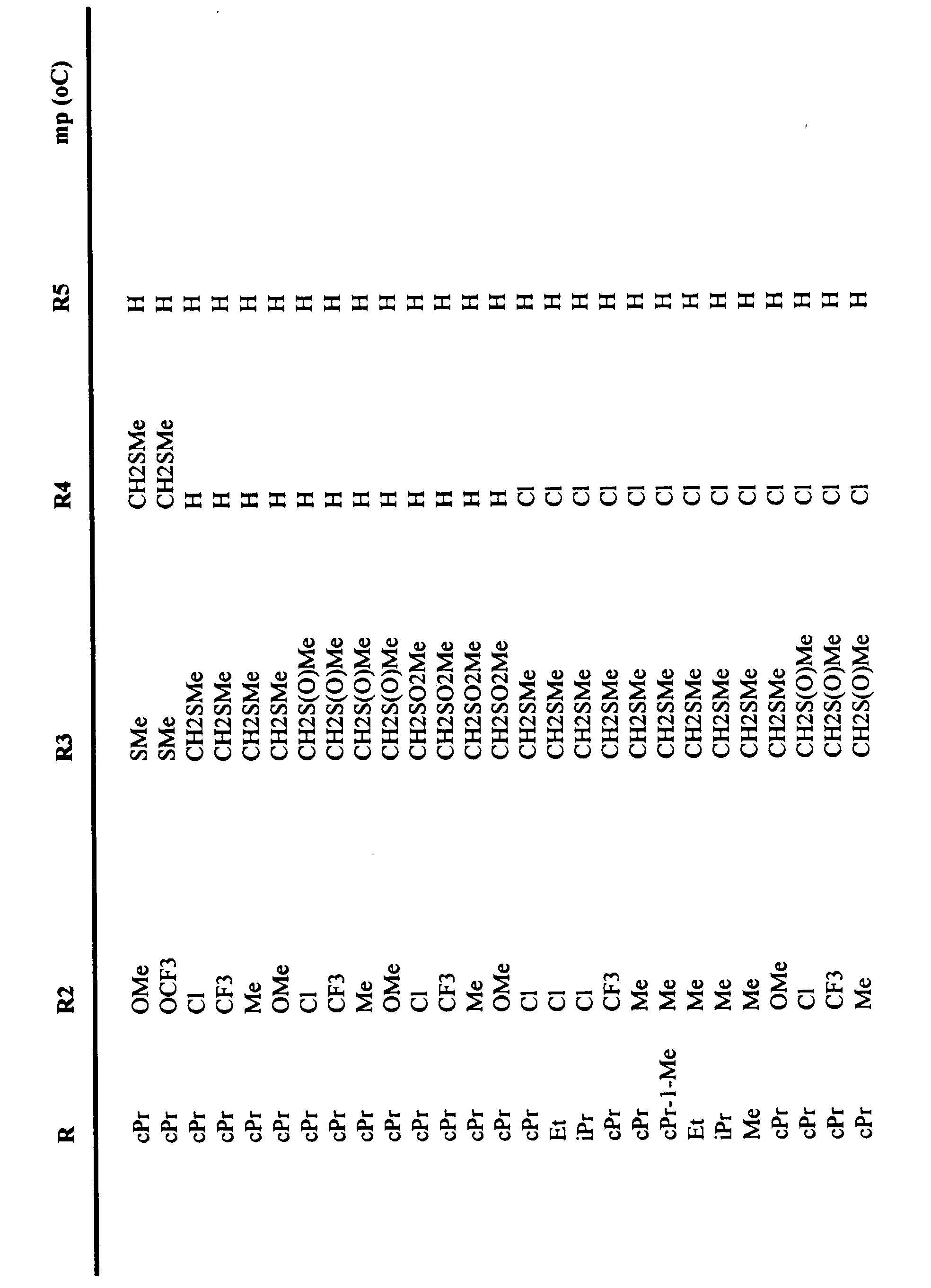

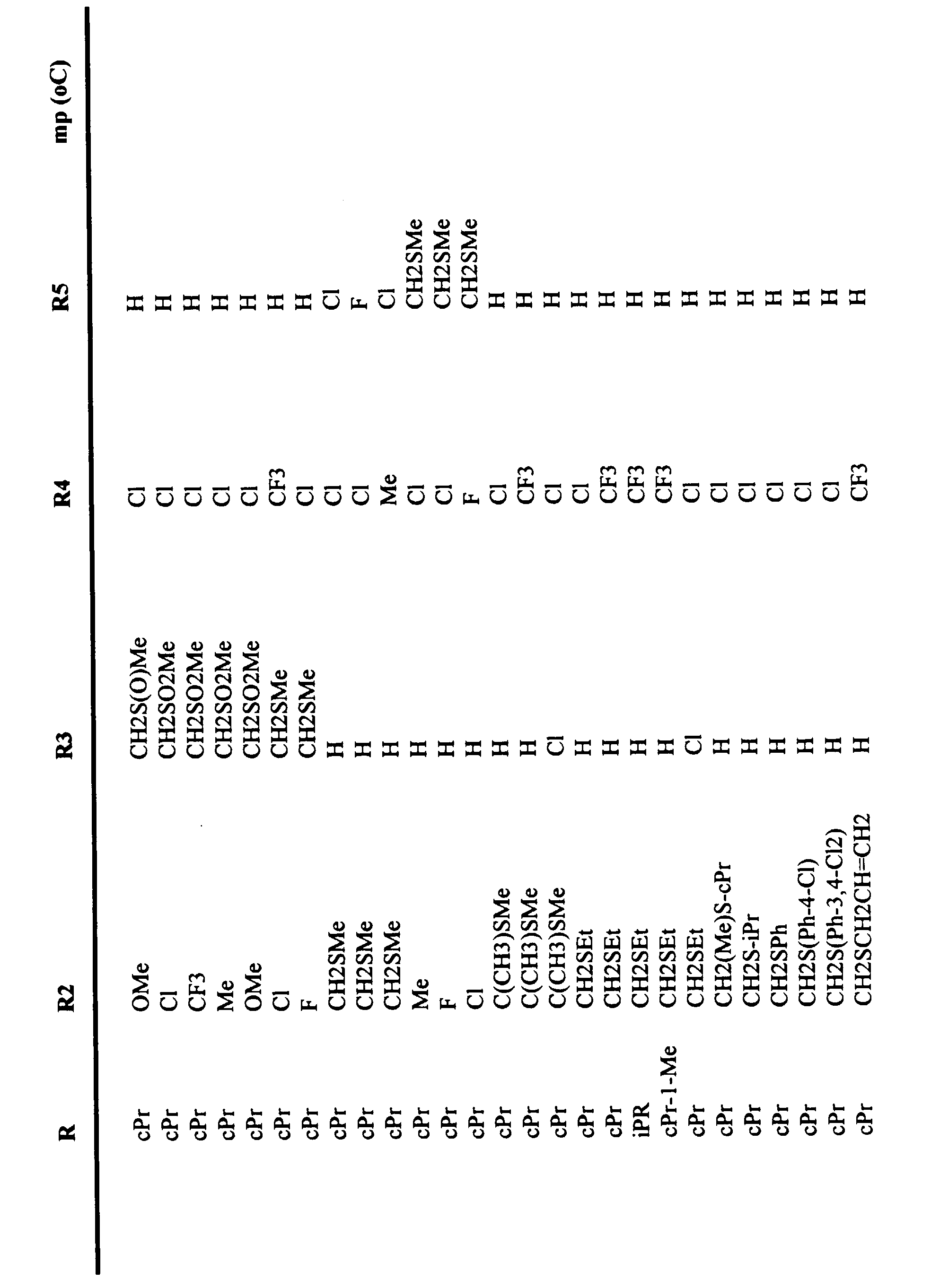

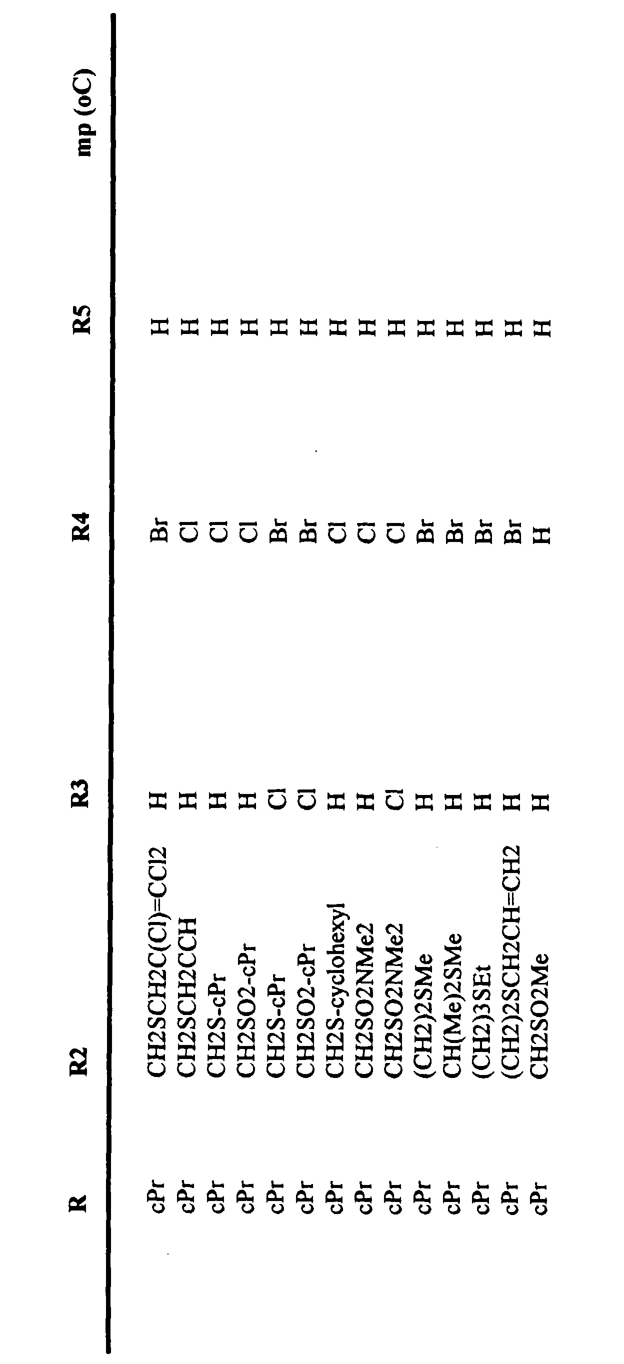

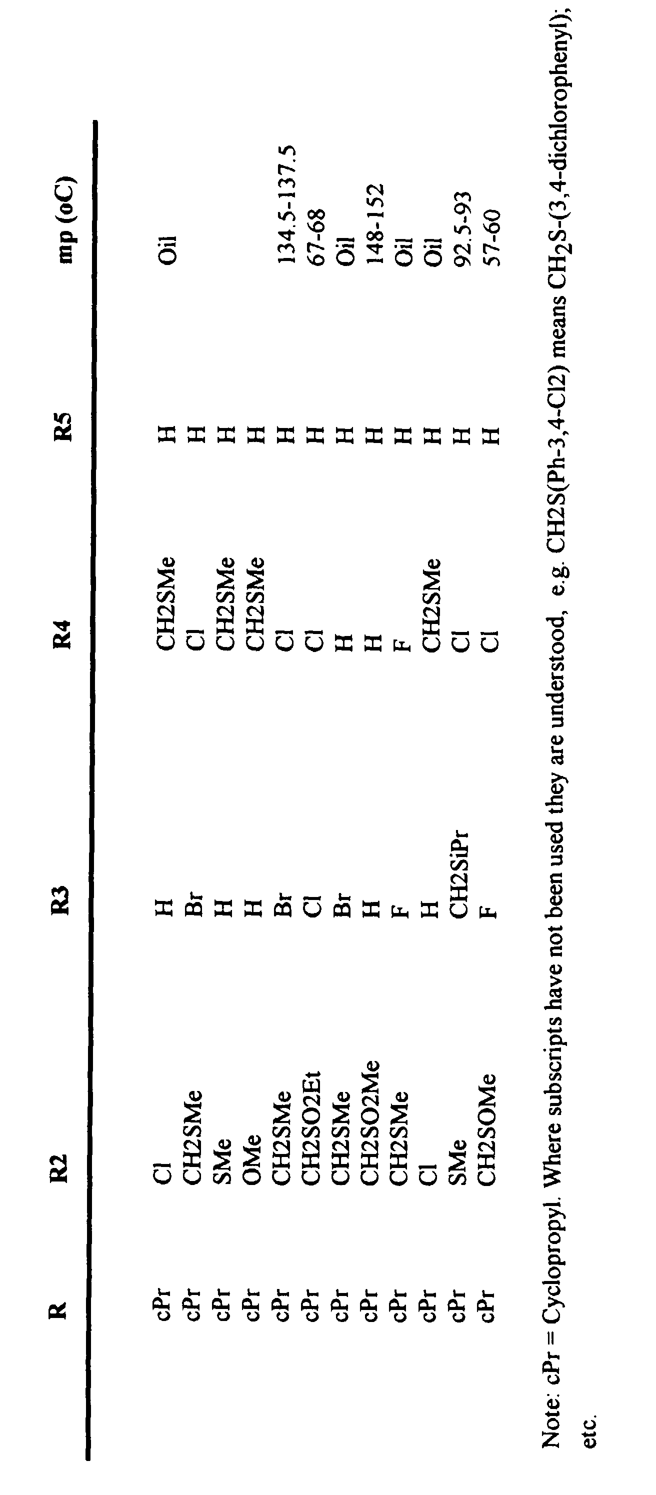

Compounds of formula (I) may be prepared by the application or adaptation of known methods (i.e. methods heretofore used or described in the literature), for example as hereinafter described.

In the following description where symbols appearing in formulae are not specifically defined, it is to be understood that they are "as hereinbefore defined" in accordance with the first definition of each symbol in the specification.

It is to be understood that in the descriptions of the following processes the sequences may be performed in different orders, and that suitable protecting groups may be required to achieve the compounds sought.

According to a feature of the present invention compounds of formula (I) may be prepared from a compound of formula (II): 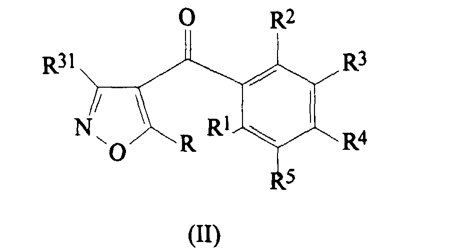 wherein R, R¹, R², R³, R⁴ and R⁵ are as hereinbefore defined and R³¹ represents the hydrogen atom or a group selected from a carboxylic ester, amide, nitrile and acyl.

Where R³¹ represents hydrogen or an acyl group the reaction is carried out by treatment with a base. Examples of suitable bases include alkali or alkaline earth metal hydroxides or alkoxides such as sodium ethoxide or organic bases such as triethylamine.

Where R³¹ represents a group such as an ester, amide or nitrile the conversion is carried out by a hydrolytic reaction. The hydrolytic reaction may be carried out in the presence of an acid or base. Acidic hydrolysis may be achieved for example using aqueous hydrochloric acid. Basic hydrolysis may be achieved for example using sodium hydroxide in a mixture of alcohol and water. The reactions are carried out at a temperature between room temperature and the reflux temperature of the mixture.

According to a further feature of the present invention compounds of formula (I) may be prepared from a compound of formula (III): 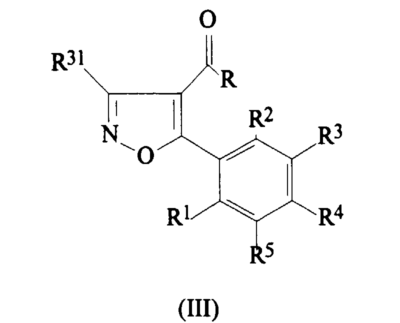 wherein R, R¹, R², R³, R⁴, R⁵ and R³¹ are as hereinbefore defined.

Where R³¹ represents hydrogen or an acyl group the reaction is carried out by treatment with a base. Examples of suitable bases include alkali or alkaline earth metal hydroxides or alkoxides such as sodium ethoxide or organic bases such as triethylamine.

Where R³¹ represents a group such as an ester, amide or nitrile the conversion is carried out by a hydrolytic reaction. The hydrolytic reaction may be carried out in the presence of an acid or base. Acidic hydrolysis may be achieved for example using aqueous hydrochloric acid. Basic hydrolysis may be achieved for example using sodium hydroxide in a mixture of alcohol and water. The reactions are carried out at a temperature between room temperature and the reflux temperature of the mixture.

According to a further feature of the present invention, compounds of formula (I) may also be prepared by the reaction of a benzoyl chloride of formula (IV): 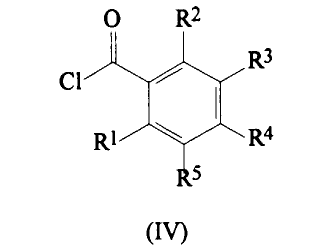 wherein R¹ R², R³, R⁴ and R⁵ are as hereinbefore defined, with a beta-ketonitrile of formula(V): 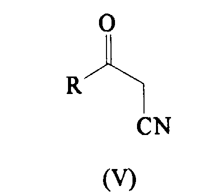 wherein R is as hereinbefore defined. The reaction is generally performed in the presence of a base in a solvent or solvent mixture. Suitable bases include metal hydrides, hydroxides or alkoxides (e.g. sodium or lithium hydride, sodium hydroxide, potassium hydroxide, magnesium ethoxide or magnesium methoxide). Suitable solvents include for example tetrahydrofuran; hydrocarbons such as toluene; or halogenated hydrocarbons such as dichloromethane. The reaction is generally performed at a temperature from 0°C to reflux temperature.

According to a further feature of the present invention, compounds of formula (I) may also be prepared by the reaction of an acid chloride of formula (VI): 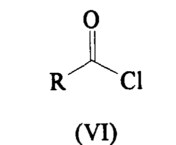 wherein R is as hereinbefore defined, with a beta-ketonitrile of formula (VII): 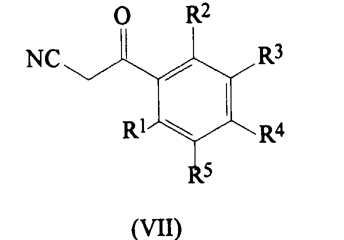 wherein R¹, R², R³, R⁴ and R⁵ are as hereinbefore defined. The reaction is generally performed in the presence of a base in a solvent or solvent mixture. Suitable bases include metal hydrides, hydroxides or alkoxides (e.g. sodium or lithium hydride, sodium hydroxide, potassium hydroxide, magnesium ethoxide or magnesium methoxide). Suitable solvents include for example tetrahydrofuran; hydrocarbons such as toluene; or halogenated hydrocarbons such as dichloromethane. The reaction is generally performed at a temperature from 0°C to reflux temperature.

According to a further feature of the present invention compounds of formula (I) may be prepared by the reaction of a benzoyl chloride of formula (IV) wherein R¹, R², R³, R⁴ and R⁵ are as hereinbefore defined, with a beta-ketonitrile of formula (V) wherein R is as hereinbefore defined, via an intermediate of formula (VIII): 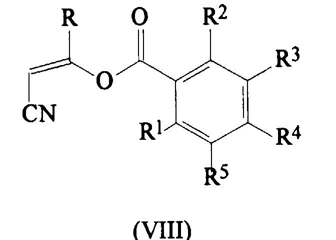 wherein R, R¹, R², R³, R⁴ and R⁵ are as hereinbefore defined. The formation of the intermediate of formula (VIII) may be carried out in the presence of a mild base such as an organic base e.g. triethylamine, in an inert solvent such as acetonitrile or dichloromethane at a temperature between room temperature and the reflux temperature of the mixture. The rearrangement of the intermediate of formula (VIII) to a compound of formula (I) may be carried out optionally in situ in an inert solvent such as acetonitrile or dichloromethane in the presence of a catalyst such as a source of cyanide. Examples of such sources of cyanide are acetone cyanhydrin or an alkali metal cyanide such as potassium cyanide, optionally in the presence of a crown ether such as 18-crown-6.

According to a further feature of the present invention compounds of formula (I) may be prepared by the reaction of an acid chloride of formula (VI) wherein R is as hereinbefore defined, with a beta-ketonitrile of formula (VII) wherein R¹, R², R³, R⁴ and R⁵ are as hereinbefore defined, via an intermediate of formula (IX): 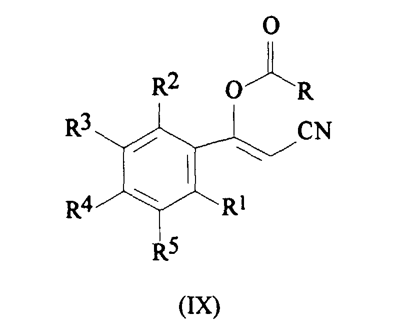 wherein R, R¹, R², R³, R⁴ and R⁵ are as hereinbefore defined. The formation of the intermediate of formula (IX) may be carried out in the presence of a mild base such as an organic base e.g. triethylamine, in an inert solvent such as acetonitrile or dichloromethane at a temperature between room temperature and the reflux temperature of the mixture. The rearrangement of the intermediate of formula (IX) to a compound of formula (I) may be carried out optionally in situ in an inert solvent such as acetonitrile or dichloromethane in the presence of a catalyst such as a source of cyanide. Examples of such sources of cyanide are acetone cyanhydrin or an alkali metal cyanide such as potassium cyanide, optionally in the presence of a crown ether such as 18-crown-6.

Intermediates in the preparation of compounds of formula (I) may be prepared by the application or adaptation of known methods.

Compounds of formula (II) or (III) in which R³¹ represents hydrogen may be prepared by the reaction of a compound of formula (X): 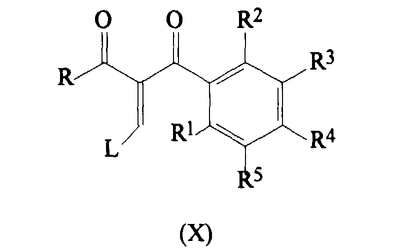 wherein R¹, R^{2,} R³, R⁴ and R⁵ are as hereinbefore defined and L is -OR⁷² or -N(R⁷²)₂ and R⁷² is an alkyl group, with a salt of hydroxylamine in the presence of a base or acid acceptor. The reaction is generally carried out using hydroxylamine hydrochloride in the presence of sodium acetate or an organic base such as triethylamine. The reaction is preferably performed in a solvent. Suitable solvents include alcohols such as ethanol or inert solvents such as acetonitrile. The reaction is carried out at a temperature between room temperature and the boiling point of the solvent.

Compounds of formula (X) in which L represents -OR⁷² may be prepared by the reaction of a diketone of formula (XI): 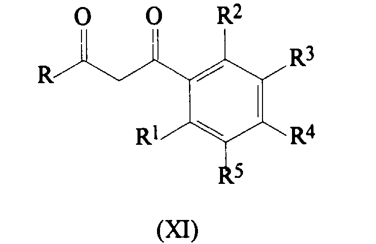 wherein R, R¹, R², R³, R⁴ and R⁵ are as hereinbefore defined, with an ortho ester, HC(OR⁷²)₃. The reaction is generally carried out using triethyl orthoformate in the presence of an acid catalyst such as acetic anhydride. The reaction is carried out at a temperature between room temperature and the boiling point of the mixture.

Compounds of formula (X) in which L represents -N(R⁷²)₂ may be prepared by the reaction of a diketone of formula (XI) with an amide acetal of formula (R⁷²)₂N-CH(OR⁷²)₂. The reaction is optionally carried out in an inert solvent such as toluene at a temperature between room temperature and the boiling point of the mixture.

Compounds of formula (II) wherein R³¹ represents an ester, nitrile or acyl group may be prepared by the reaction of a compound of formula (XII): 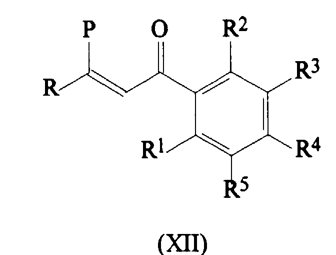 wherein R, R¹, R^{2,} R³, R⁴ and R⁵ are as hereinbefore defined and P is a leaving group such as N,N-dialkylamino, with a compound of formula R³¹-C(Z)=NOH wherein R³¹ represents an ester, nitrile or acyl group and Z is a halogen atom. Generally Z is chlorine or bromine atom. The reaction is generally performed in an inert solvent such as toluene or dichloromethane either in the presence of a base such as triethylamine or a catalyst such as a 4 Angstrom molecular sieve or fluoride ion.

Compounds of formula (XII) may be prepared by the reaction of a compound of formula CH₂=C(R³¹)(P), wherein R³¹ and P are as hereinbefore defined, with a benzoyl chloride of formula (IV). The reaction is generally carried out in the presence of an organic base such as triethylamine in an inert solvent such as toluene or dichloromethane at a temperature between -20°C and room temperature.

Compounds of formula (II) or (III) wherein R³¹ represents an ester, nitrile or acyl group may be prepared by the reaction of a compound of formula (XI) with a compound of formula R³¹-C(Z)=NOH wherein R³¹ represents an ester, nitrile or acyl group and Z is as hereinbefore defined. Generally Z is a chlorine or bromine atom. The reaction is generally performed in an inert solvent such as dichloromethane or acetonitrile in the presence of a base. Examples of suitable bases are alkaline earth metal alkoxides such as magnesium methoxide and the reaction is carried out a temperature between room temperature and the reflux temperature of the mixture.

Compounds of formula (II) or (III) wherein R³¹ represents an amide group may be prepared by the reaction of the corresponding compound of formula (II) or (III) in which R³¹ represents an ester group with ammonia or an amine. The reaction is carried out in a solvent or solvent mixture such as aqueous ethanol at a temperature between room temperature and the reflux temperature of the mixture.

Compounds of formula (III) in which R³¹ represents hydrogen may be prepared by the reaction of a compound of formula (XIII): 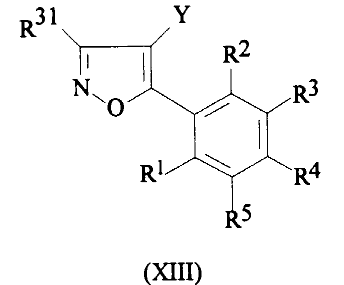 in which R³¹ represents hydrogen and Y represents a carboxy group, or a reactive derivative thereof (such as a carboxylic acid chloride or carboxylic ester) or a cyano group, with an appropriate organometallic reagent such as a Grignard reagent or an organolithium reagent, to introduce the group -COR into the 4-position of the isoxazole ring. The reaction is generally carried out in an inert solvent such as ether or tetrahydrofuran, at a temperature from 0°C to the reflux temperature of the solvent.

Compounds of formula (III) in which R³¹ is an ester, nitrile or acyl group may be prepared by the reaction of a compound of formula (XIV): 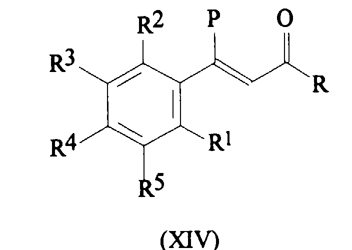 wherein P is a leaving group such as N,N-dialkylamine with a compound of formula R³¹C(Z)=N-OH wherein Z is as hereinbefore defined and R³¹ is an ester, nitrile or acyl group. Generally Z is chlorine or bromine. The reaction is generally performed in an inert solvent such as toluene or dichloromethane either in the presence of a base such as triethylamine or a catalyst such as a 4A molecular sieve or fluoride ion.

Compounds of formula (XIII) in which R³¹ is a hydrogen atom and Y is -CO₂-alkyl or -CN may be prepared by the reaction of a compound of formula (XV): 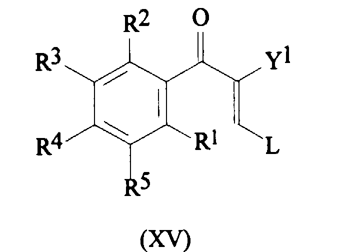 wherein Y¹ represents CO₂-alkyl or -CN and L is as hereinbefore described, with a salt of hydroxylamine such as hydroxylame hydrochloride, in a solvent such as ethanol or acetonitrile, optionally in the presence of a base or acid acceptor such as triethylamine or sodium acetate.

Compounds of formula (XIII) in which R³¹ represents hydrogen and Y represents a carboxylic acid or carboxylic acid chloride may be prepared from the corresponding compound of formula (XIII) in which R³¹ represents hydrogen and Y represents a carboxylic ester group by the hydrolysis of said ester group and conversion, as necessary, of the acid thus obtained to the acid chloride, e.g. by heating with thionyl chloride.

Compounds of formula (XV) may be prepared by the reaction of a compound of formula (VII) or a ketoester of formula (XVI): 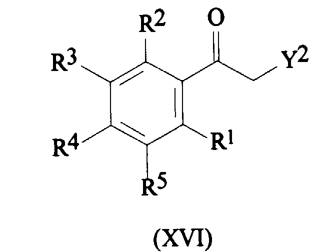 wherein R¹, R², R³, R⁴ and R⁵ are as hereinbefore defined and Y² represents -CO₂-alkyl, with either triethyl orthoformate in the presence of acetic anhydride at the reflux temperature of the mixture or with dimethylformamide dimethylacetal optionally in an inert solvent such as toluene at a temperature from room temperature to the reflux temperature of the mixture.

Compounds of formula (XIV) may be prepared by the reaction of a compound of formula (XVII) : 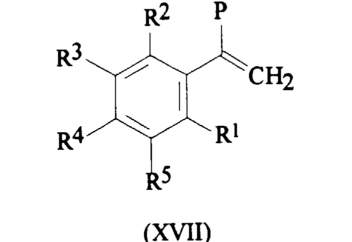 wherein R¹, R², R³, R⁴, R⁵ and P is as hereinbefore defined, with an acid chloride of formula (VI) wherein R is as hereinbefore defined, in an inert solvent such as dichloromethane or toluene, in the presence of a base such as triethylamine.

Acid chlorides of formula (IV) or (VI) are generally known or can be prepared from the corresponding carboxylic acid according to commonly accepted methods, for example by using thionyl chloride in chloroform at reflux.

Beta-ketonitriles of formula (V) may be prepared from acid chlorides of formula (VI) by a number of methods well known in the chemical literature. For example, see Krauss, et al, Synthesis, **1983**, 308, or Muth, et al, J. Org. Chem, **1960**, 25, 736. Alternatively beta-ketonitriles of formula (V) may be prepared by the reaction of an ester of formula R-CO₂Et , wherein R is as hereinbefore defined, with acetonitrile. This reaction is described in the literature for example see the article by Abramovitch and Hauser, J. Am. Chem. Soc., **1942**, 64, 2720.

Beta-ketonitriles of formula (VII) may be prepared from benzoyl chlorides of formula (IV) or from ethyl benzoates of formula (XVIII): 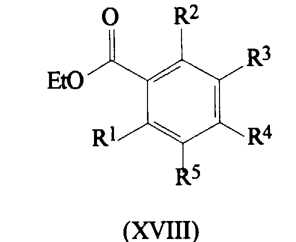 wherein R¹, R², R³, R⁴ and R⁵ are as hereinbefore defined, in a manner analogous to the preparation of beta-ketonitriles of formula (V) set forth above.

Compounds of formula (V), (XI), (XIV), (XVI), (XVII) and (XVIII) are known or may be prepared by the application and adaptation of known methods.

Agriculturally acceptable salts and metal complexes of compounds of formula (I) may be prepared by known methods.

The following examples illustrate the preparation of compounds of general formula (I). In the present specification b.p. means boiling point, m.p. means melting point. Where the letters NMR appear, the characteristics of the proton nuclear magnetic resonance spectrum follow. In the following description cPr represents cyclopropyl; CHex represents cyclohexyl. Unless otherwise specified the percentages are by weight.

It is understood that for preparing the compounds of the invention listed below, certain examples and reference examples are included only for reasons of illustration of the methods employed.

### EXAMPLE 1

Sodium metal (0.11g) was dissolved in absolute ethanol with stirring and was cooled to ambient temperature. 4-(2-Cyclohexylsulphonyl-4-trifluoromethybenzoyl)-5-cyclopropylisoxazole (1.5g) in absolute ethanol was added and the resulting solution was stirred at ambient temperature for 3 hours. It was poured into ice/water, acidified to pH1 with concentrated hydrochloric acid and the resultant white suspension was allowed to warm to ambient temperature before it was extracted with ethyl acetate. The combined organic extracts were washed with water and dried (magnesium sulphate). Evaporation of the solvent gave a light-brown solid (1.3g) of 2-cyano-1-(2-cyclohexylsulphonyl-4-trifluoromethylphenyl)-3-cyclopropylpropan-1,3-dione m.p. 60-62°C.

By proceeding in a similar manner the following compounds of formula (I) were prepared from the appropriately substituted starting materials.

| **R** | **R**^{**1**} | **R**^{**2**} | **R**^{**3**} | **R**^{**4**} | **R**^{**5**} | **mp(°C) or NMR** |
|---|---|---|---|---|---|---|
| cPr | H | CH₂SMe | Br | Cl | H | 134.5-137.5 |
| cPr | H | CH₂SO₂Et | Cl | Cl | H | 64 -68 |
| cPr | H | CH₂SMe | Br | H | H | (a) |

| | | | | | | |
|---|---|---|---|---|---|---|
| (a) NMR(CDCl₃): 1.2(m,2H), 1.35(m,2H), 2.0(s,3H), 2.3(m,1H), 4.0(s,2H), 7.15(m,H), 7.4(dd,1H), 7 65(dd,1H), 17.3(bs, 1H). | | | | | | |

### EXAMPLE 2

4-(2-Methylsulphonylmethyl-4-bromobenzoyl)-5-cyclopropylisoxazole (0.3g) in dichloromethane was treated at ambient temperature with triethylamine (0.124 ml) and the reaction mixture was stirred at ambient temperature for 3.33 hours. It was treated with 2M hydrochloric acid, diluted with dichloromethane and brine, extracted with more dichloromethane and dried (sodium sulphate). Evaporation of solvent gave a beige gum (0.3g). This was triturated with diisopropyl ether to give 2-cyano-3-cyclopropyl-1-(2-methylsulphonylmethyl-4-bromophenyl)-propan-1,3-dione as a white solid (0.24g), m.p. 108°C.

By proceeding in a similar manner the following compounds of formula (I) were prepared from the appropriately substituted starting materials.

| **R** | **R**^{**1**} | **R**^{**2**} | **R**^{**3**} | **R**^{**4**} | **R**^{**5**} | **m.p. (°C)/NMR** |
|---|---|---|---|---|---|---|
| cPr | H | CH₂SO₇Me | H | H | H | 148-152 |
| cPr | H | CH₂SMe | F | F | H | (a) |
| cPr | H | Cl | H | CH₂SMe | H | (b) |
| cPr | H | SMe | CH₂SPrⁱ | Cl | H | 92.5-93 |

| | | | | | | |
|---|---|---|---|---|---|---|
| (a) NMR(CDCl₃) 1.23(m,2H), 1.38(m,2H), 1.97(s,3H), 2.33(m,1H), 3.88(s,2H), 5.22(s, enol CH), 7.1(m,1H), 7.32(m,1H). | | | | | | |
| (b) NMR(CDCl₃) 1.23(m,2H), 1.38(m,2H), 1.92(s,3H), 2.32(m,1H), 3.60(s,2H), 7.26(m,1H), 7.40(m,1H), 17.4(brs,1H). | | | | | | |

### REFERENCE EXAMPLE 1

A mixture of 5-cyclopropyl-4-(2-phenylsulphenyl-4-trifluoromethyl)benzoylisoxazole (1g) and 3-chloroperoxybenzoic acid (1g) in dichloromethane was stirred at room temperature for 1 hour. The precipitate was filtered and the filtrate washed with aqueous sodium metabisulphite, aqueous sodium bicarbonate, dried over anhydrous sodium sulphate and filtered. The dichloromethane was evaporated to give 5-cyclopropyl-4-(2-phenylsulphonyl-4-trifluoromethyl)-benzoylisoxazole as a white solid, m.p. 174°C.

By proceeding in a similar manner the following compound of formula (II) above was prepared from the appropriately substituted starting materials.

| **R**^{**31**} | **R** | **R**^{**1**} | **R**^{**2**} | **R**^{**3**} | **R**^{**4**} | **R**^{**5**} | **m.p.(°C)** |
|---|---|---|---|---|---|---|---|
| H | cPr | H | CH₂SO₂Me | H | Br | H | 153.6-154.8 |

### REFERENCE EXAMPLE 1(a)

3-Chloroperbenzoic acid (50%, 1.35g) was added to a solution of 5-cyclopropyl-4-[3,4-dichloro-2-(ethylsulphenylmethyl)benzoyl]-isoxazole (0.7g) in dichloromethane (30ml) at -20°C and then stirred at room temperature for 2 hours. The mixture was washed with sodium metabisulphite, then with sodium bicarbonate and with water, dried (magnesium sulphate) and evaporated to give 5-cyclopropyl-4-[3,4-dichloro-2-(ethylsulphonylmethyl)-benzoyl]isoxazole (0.51 g), m.p. 152.5-153.5°C.

By proceeding in a similar manner 5-cyclopropyl-4-(2-methylsulphonylmethylbenzoyl)isoxazole was prepared from the appropriately substituted starting materials, mp. 131.5-133.5°C.

### REFERENCE EXAMPLE 2

A mixture of 3-cyclopropyl-2-ethoxymethylene-1-(2-phenylsulphenyl-4-trifluoromethylphenyl)propan-1,3-dione (7g), hydroxylamine hydrochloride (1.24g) and sodium acetate (1.5g) in ethanol was stirred at 25°C for 2 hours. The mixture was then poured into water and extracted with ethyl acetate. The solution was dried over anhydrous sodium sulphate and filtered. The filtrate was evaporated and the residue purified by column chromatography on silica, using a mixture of ethyl acetate and hexane as eluent. The resulting solution was evaporated and the residue crystallised from cyclohexane to give 3.06g of 5-cyclopropyl-4-(2-phenylsulphenyl-4-trifluoromethylbenzoyl)isoxazole, m.p. 115°C.

By proceeding in a similar manner the following compounds of formula (II) were prepared from the appropriately substituted starting materials.

| **R**^{**31**} | **R** | **R**^{**1**} | **R**^{**2**} | **R**^{**3**} | **R**^{**4**} | **R**^{**5**} | **m.p.(°C) or NMR** |
|---|---|---|---|---|---|---|---|
| H | cPr | H | CH₂SMe | H | Br | H | 84.2-85.7 |
| H | cPr | H | CH₂SMe | Br | Cl | H | a |
| H | cPr | H | CH₂SEt | Cl | Cl | H | 77.5-78 |
| H | cPr | H | CH₂SMe | Br | H | H | b |
| H | cPr | H | SMe | CH₂SPrⁱ | Cl | H | c |

| | | | | | | | |
|---|---|---|---|---|---|---|---|
| a NMR(CDCl₃)1.2-1.45(m,4H), 2.05(s,3H), 2.6(m,1H), 4.2(s,2H), 7.25(d,1H), 7.45(d,1H), 8.2(s,1H). | | | | | | | |
| b NMR(CDCl₃) 1.1-1.4(m,4H), 2.05(s,3H), 2.6(m,1H), 4.1(s,2H), 7.2(dd,1H), 7.3(d,1H), 7.7(d,1H), 8.2(s,1H). | | | | | | | |
| c NMR(CDCl₃)1.18(m,2H), 1.31(m,2H), 1.36(d,6H), 2.37(s,3H), 3.12(m,1H), 4.26(s,2H), 7.12(d,1H), 7.48(d,1H), 8.16(s,1H). | | | | | | | |

### REFERENCE EXAMPLE 3

Hydroxylamine hydrochloride (0.76g) was added to a mixture of 1-[4-chloro-2-(N-ethyl-N-methylsulphonylamino)phenyl]-3-cyclopropyl-2dimethylaminomethylenepropan-1,3-dione(3.83g) in ethanol. The mixture was stirred for 1 hour and evaporated to dryness. The residue was dissolved in dichloromethane and washed with water, dried (MgSO₄) and filtered. The filtrate was evaporated to dryness and the residue was purified by chromatography eluted with a mixture of cyclohexane, dichloromethane and ethyl acetate. The product was triturated with a mixture of ether and hexane and filtered to give 4-[4-chloro-2-(N-ethyl-N-methylsulphonylamino)benzoyl]-5-cyclopropylisoxazole (0.81g) as a white solid, m.p. 114-115.8°C.

By proceeding in a similar manner the following compounds of formula (II) above were prepared from the appropriately substituted starting materials.

| **R**^{**31**} | **R** | **R**^{**1**} | **R**^{**2**} | **R**^{**3**} | **R**^{**4**} | **R**^{**5**} | **m.p.(°C) or NMR** |
|---|---|---|---|---|---|---|---|
| H | cPr | H | CH₂SMe | H | Br | H | 84.2-85.6 |
| H | cPr | H | CH₂SMe | H | H | H | (a) |
| H | cPr | H | CH₂SMe | F | F | H | (b) |
| H | cPr | H | Cl | H | CH₂SMe | H | 58-61 |

| | | | | | | | |
|---|---|---|---|---|---|---|---|
| (a) NMR(CDCl₃)1.11(m,2H), 1.25(m,2H), 1.9(s,3H), 2.5(m,1H), 3.82(s,2H), 7.33(m,4H), 8.17(s,1H). | | | | | | | |
| (b) NMR(CDCl₃) 1.14(m,2H), 1.20(m,2H), 1.90(s,3H), 2.26(m,1H), 3.85(s,2H), 7.05(m,1H), 7.25(m,1H), 8.10(s,1H) | | | | | | | |

### REFERENCE EXAMPLE 4

A mixture of 3-cyclopropyl-1-(2-phenylsulphenyl-4-trifluoromethylphenyl)propan-1,3-dione (6.0g) and triethylorthoformate (4.9g) in acetic anhydride was stirred and heated at reflux for 3 hours. It was cooled and evaporated. The residue was treated with toluene and re-evaporated to dryness to give 1-cyclopropyl-2-ethoxymethylene-3-(2-phenylsulphenyl-4-trifluoromethylphenyl)propan-1,3-dione (6.7g) as a red oil.

By proceeding in a similar manner the following compounds of the formula (X) above in which L represents ethoxy were prepared from the appropriately substituted starting materials.

| **R** | **R**^{**1**} | **R**^{**2**} | **R**^{**3**} | **R**^{**4**} | **R**^{**5**} |
|---|---|---|---|---|---|
| cPr | H | CH₂SMe | Br | Cl | H |
| cPr | H | CH₂SEt | Cl | Cl | H |
| cPr | H | CH₂SME | Br | H | H |
| cPr | H | SMe | CH₂SPrⁱ | Cl | H |

### REFERENCE EXAMPLE 5

A mixture of 1-[4-chloro-2-(N-ethyl-N-methyl-sulphonylamino)-phenyl]-3-cyclopropylpropan-1,3-dione (3.5g) and dimethylformamide dimethyl acetal (1.5ml) in dichloromethane was stirred at room temperature overnight and heated at reflux for 3 days. After cooling the mixture was evaporated to dryness to give 1-[4-chloro-2-(N-ethyl-N-methylsulphonylaminophenyl]-3-cyclopropyl-2-dimethylaminomethylenepropan-1,3-dione (3.83g) as an orange gum.

By proceeding in a similar manner the following compounds of the formula (X) above in which L represents N,N-dimethylamino were prepared from the appropriately substituted starting materials.

| **R** | **R**^{**1**} | **R**^{**2**} | **R**^{**3**} | **R**^{**4**} | **R**^{**5**} |
|---|---|---|---|---|---|
| cPr | H | CH₂SMe | H | Br | H |
| cPr | H | CH₂SMe | H | H | H* |
| cPr | H | CH₂SMe | F | F | H* |
| cPr | H | Cl | H | CH₂SMe | H* |

| | | | | | |
|---|---|---|---|---|---|
| * Reaction performed in toluene | | | | | |

### REFERENCE EXAMPLE 6

A solution of methyl (2-phenylsulphenyl-4-trifluoromethyl)-benzoate (8g) and cyclopropyl methyl ketone (4.2g) in tetrahydrofuran was added to a suspension of sodium hydride (1.65g of 80% sodium hydride in oil) in tetrahydrofuran at 60°C. After the addition was complete the temperature was kept at 60°C for a further 15 minutes. The mixture was then cooled to 25°C and poured into water. An aqueous solution of hydrochloric acid was added to the mixture to pH 1. The mixture was extracted with ethyl acetate, dried over anhydrous sodium sulphate, filtered and evaporated. The residue was purified by chromatography on silica, using ethyl acetate as eluent to give, after evaporation of the solvent, 3-cyclopropyl-1-(2-phenylsulphenyl-4-trifluoromethylphenyl)-propan-1,3-dione as a red oil (8.07g), NMR (CDCl₃) 0.9(2H,m), 1.25(2H,m), 1.70(1H,m), 6.05(1H,s), 7.1(1H,s), 7.3-7.5(6H,m), 7.6(1H,s).

By proceeding in a similar manner the following compounds were prepared:
3-cyclopropyl-1-(2-methylsulphenylmethyl-4-bromophenyl)-propan-1,3-dione, NMR(CDCl₃) 0.9-11(m,4H) 1.6-1.75(m,1H) 2.05(s,3H) 3.9(s,2H) 6.0(s,1H) 7.35(d,1H) 7.45(d,1H) 7.6(s,1H) 15.9-16.2(bs,1H).
3-cyclopropyl-1-(3-bromo-4-chloro-2-methylsulphenylmethylphenyl)-propan-1,3-dione, NMR (CDCl₃) 1.05(m,2H), 1.22(m,2H), 1.75(m,1H), 2.1(s,3H), 4.2(s,2H), 6.0(s,1H), 7.35(d,1H), 7.35(d,1H), 16.0(bs,1H).
3-cyclopropyl-1-(3,4-dichloro-2-ethylsulphenylmethylphenyl)-propan-1,3-dione, m.p. 105-109°C.
1-(3-bromo-2-methylsulphenylmethylphenyl)-3-cyclopropylpropan-1,3-dione, m.p. 65-68°C.
3-cyclopropyl-1-(2-methylsulphenylmethylphenyl)propan-1,3-dione, NMR(CDCl₃) 0.95 (m,2H), 1.13(m,2H), 1.71(m,1H), 1.92(s,3H), 4.08(s,2H), 5.98(s,1H), 7.32(m,4H), 16.06(s,1H).
3-cyclopropyl-1-(3,4-difluoro-2-methylsulphenylmethylphenyl)propan-1,3-dione.
1-(2-chloro-4-methylsulphenylmethylphenyl)-3-cyclopropylpropan-1,3-dione, NMR (CDCl₃) 1.0(m,2H), 1.2(m,2H), 1.75(m,1H), 2.0(s,3H), 3.6(s,2H), 6.2(s,1H), 7.2(dd,1H), 7.4(d,1H), 7.6(d,1H), 15.9(bs,1H).

### REFERENCE EXAMPLE 7

A suspension of magnesium (0.47g) in methanol was warmed gently to initiate reaction and then heated at reflux until all of the magnesium had dissolved. It was cooled slightly and t-butyl 3-cyclopropyl-3-oxopropionate (2.39g) was added. The mixture was stirred and heated at reflux for 25 minutes then evaporated to dryness. It was dissolved in toluene and re-evaporated to dryness. The residue was again dissolved in toluene and 2-chloro-4-(methylsulphonylamino)benzoyl chloride (3.2g) was added. The mixture was stirred at room temperature overnight. Hydrochloric acid (2M) was added and the mixture was stirred. The layers were separated and the organic layer was dried (MgSO₄) and filtered. The filtrate was evaporated to give t-butyl 2-[2-chloro-4-(methylsulphonylamino)benzoyl]-3-cyclopropyl-3-oxopropionate (3.7g) as a white solid, m.p. 137-140°C.

t-Butyl 2-[2-chloro-4-(methylsulphonylamino)benzoyl]-3-cyclopropyl-3-oxopropionate (2g) was dissolved in toluene and p-toluenesulphonic acid (0.2g) was added. The mixture was stirred and heated at reflux for 0.5 hours. It was cooled, washed with water, dried (MgSO₄) and filtered. The filtrate was evaporated to dryness to give 1-[2-chloro-4-(methylsulphonylamino)phenyl]-3-cyclopropylpropan-1,3-dione (1.48g) as a white solid, m.p. 119.9-121.6°C.

By proceeding in a similar manner the following compound of formula (XI) above was prepared from the appropriately substituted starting materials without isolation of the intermediate ester.

| **R** | **R**^{**1**} | **R**^{**2**} | **R**^{**3**} | **R**^{**4**} | **R**^{**5**} | **m.p. (°C) or NMR** |
|---|---|---|---|---|---|---|
| cPr | H | SMe | CH₂SPrⁱ | Cl | H | a |

| | | | | | | |
|---|---|---|---|---|---|---|
| a NMR(CDCl₃) 1.0(m,2H), 1.20(m,2H), 1.32(d,6H), 1.72(m,1H), 2.4(s,3H), 3.12(m,1H), 4.3(s,2H), 7.28(m, 1H), 7.39(m,1H). Note - The corresponding reaction between cyclopropyl methylketone and methyl 4-chloro-2-methylsulphenyl-3-isopropylsulphenylmethyl benzoate gave 4-chloro-2-methylsulphenyl-3-isopropyl-sulphenylmethylbenzoic acid, NMR(CDCl₃) 1.22(m,2H), 1.32(m,2H), 1.35(d,6H), 2.50(s,3H), 3.12(m,1H), 7.46(d,1H), 7.79(d,1H) | | | | | | |

Benzoyl chlorides were prepared by heating the appropriately substituted benzoic acids with thionyl chloride. The excess thionyl chloride was removed by evaporation the benzoyl chlorides thus obtained were used without further purification.

### REFERENCE EXAMPLE 8

Concentrated sulphuric acid (20 ml) was added to a suspension of 2-chloro-4-(N-methylsulphonylamino)benzoic acid (10.3g) in methanol and the mixture was stirred and heated at reflux for 22 hours. It was cooled, evaporated to dryness and diluted with water, extracted with ethyl acetate, washed with aqueous sodium bicarbonate solution, water, dried (MgSO₄) and filtered. The filtrate was evaporated to dryness to give methyl 2-chloro-4(N-methylsulphonylamino)benzoate(10.0g) as an off-white solid, NMR (DMSO-d₆) 3.15(s,3H) 3.85(s,3H) 7.2(d,1H) 7.3(s,1H) 7.85(d,1H) 10.5(s,1H).

By proceeding in a similar manner the following compound was prepared:
methyl 3,4-difluoro-2-methylbenzoate, m.p. 44-45.8°C.

### REFERENCE EXAMPLE 9

A mixture of methyl 4-bromo-2-(bromomethyl)benzoate (12g) and sodium thiomethoxide (2.5g) in toluene was stirred at 100°C for 2 hours. The mixture was then cooled, poured into water and extracted with ethyl acetate. The organic layer was separated, washed with brine, dried (anhydrous sodium sulphate) and evaporated to afford a brown oil which was purified by column chromatography on silica gel giving methyl 4-bromo-2-(methylsulphenylmethyl)benzoate (4.1g) as white crystals, m.p. 79.3°C.

By proceeding in a similar manner the follow compound was prepared:
methyl 2-chloro-4-methylsulphenylmethylbenzoate, NMR(CDCl₃) 1.9(s,3H), 3.6(s,2H), 3.9(s,3H), 7.2(dd,1H), 7.35(d,1H), 7.7(d,1H).

### REFERENCE EXAMPLE 10

Isoamyl nitrite (150ml) was added to a stirred mixture of 4-(N,N-dimethylaminosulphonyl)-2-trifluoromethylbenzamide (15.1g) in acetic acid and concentrated sulphuric acid while maintaining the temperature at 70-80°C. It was cooled, poured into water and extracted with ethyl acetate, washed with water, dried (MgSO₄) and filtered to give 4-(N,N-dimethylaminosulphonyl)-2-trifluoromethylbenzoic acid (11.2g) as a white solid, m.p. 188.9-189.4°C.

By proceeding in a similar manner the following compound was prepared:
methyl 2-chloro-4-methylsulphenylmethylbenzoate, NMR(CDCl₃) 1.9(s,3H), 3.6(s,2H), 3.9(s,3H), 7.2(dd,1H), 7.35(d,1H), 7.7(d,1H).

### REFERENCE EXAMPLE 11

Oxalyl chloride (2.3ml) was added to a stirred solution of 2-(N-methyl-N-methylsulphonylamino)-4-trifluoromethoxybenzoic acid (6.2g) in 1,2-dichloroethane, N,N-Dimethylformamide (3 drops) was added and stirring maintained for 2 hours. After evaporation, 2-(N-methyl-N-methylsulphonylamino)-4-trifluoromethoxybenzoyl chloride (6.3g) was obtained as a red oil and used directly in the next stage.

By proceeding in a similar manner the following compound was prepared:
4-chloro-2-methylsulphenyl-3-isopropylsulphenylmethyl benzoyl chloride;

### REFERENCE EXAMPLE 12

Sodium thiomethoxide (5.14g) was added to a mixture of ethyl 3-bromo-2-bromomethyl-4-chlorobenzoate (20.14g) and tetraethylammonium iodide (0.5g) in tetrahydrofuran (200ml) and stirred overnight at room temperature. Water was added, the mixture extracted (ether) and the organic phase washed (water), dried (magnesium sulphate) and evaporated to give ethyl 3-bromo-4-chloro-2-methylsulphenylmethylbenzoate (14.6g) as a brown oil, NMR(CDCl₃) 1.25(t,3H), 2.0(s,3H), 4.25(q,2H), 4.3(s,2H), 7.5(m,2H).

By proceeding in a similar manner the following compounds were prepared:
ethyl 3,4-dichloro-2-ethylsulphenylmethylbenzoate, NMR(CDCl₃) 1.2(t,3H), 1.35(t,3H), 2.5(q,2H), 4.35(q,2H), 4.35(s,2H), 7.55(m,2H);
ethyl 3-bromo-2-methyisuiphenyimethyibenzoate, NMR(CDCl₃) 1.45(t,3H), 2.0(s,3H), 4.3(q,2H), 7.1(dd,1H), 7.6(d,1H), 7.7(d,1H);
methyl 4-chloro-2-methylsulphenyl-3 -isopropylsulphenylmethyl benzoate, NMR(CDCl₃) 1.32(d,6H), 2.48(s,3H), 3.12(m,1H), 3.94(s,3H), 4.30(s,2H), 7.33(d,1H), 7.39(d,1H), from methyl 4-chloro-2-fluoro-4.30(s,2H), 7.33(d,1H), 7.39(d,1H) from methyl 4-chloro-2-fluoro-3 -isopropylsulphenylmethylbenzoate;
methyl 4-chloro-2-fluoro-3-isopropylsulphenylmethylbenzoate used directly in the next stage;

By proceeding in a similar manner but in the absence of the tetraethylammonium iodide the following compounds were prepared:
methyl 2-methylsulphenylmethylbenzoate, NMR(CDCl₃) 1.92(s,3H), 3.,8(s,3H), 4.04(s,2H), 7.25(m,2h), 7.37(dd,1H), 7.84(dd,1H).
methyl 3,4-difluoro-2-methylsulphenylmethylbenzoate, m.p. 39.5-40°C.

### REFERENCE EXAMPLE 13

A mixture of ethyl 3-bromo-4-chloro-2-methylbenzoate (3.5g) and N-bromosuccinimide (2.8g) in carbon tetrachloride (40ml) was irradiated with u.v. light in a photochemical reactor for 4.5 hours. The cooled mixture was filtered, washed with water, dried (magnesium sulphate) and evaporated to give ethyl 3 -bromo-2-bromomethyl-4-chlorobenzoate (4.7g) as an orange oil, NMR(CDCl₃) 1.35(t,3H), 4.35(q,2H), 5.15(s,2H), 7.6(m,2H).

By proceeding in a similar manner the following compounds were prepared:
ethyl 2-bromomethyl-3,4-dichlorobenzoate, NMR(CDCl₃) 1.45(t,3H), 4.4(q,2H), 5.15(s.2H), 7.7(dd,2H);
ethyl 3-bromo-2-bromomethylbenzoate, NMR(CDCl₃) 1.4(t,3H), 4.35(q,2H), 7.15(dd,1H), 7.7(d,1H), 7.8(d,1H);
methyl 2-bromomethyl-3,4-difluorobenzoate, NMR(CDCl₃) 3.97(s,3H), 5.00(s,2H), 7.20(m,1H), 7.80(m,1H);
methyl 4-bromomethyl-2-chlorobenzoate, NMR(CDCl₃) 3.9(s,3H), 4.45(s,2H), 7.3(dd,1H), 7.5(d,1H), 7.8(d,1H);
methyl 3-bromomethyl-4-chloro-2-fluorobenzoate, NMR(CDCl₃) 3.90(s,3H), 4.58(s,2H), 7.21(m,1H), 7.80(m,1H).

### REFERENCE EXAMPLE 14

N-Chlorosuccinimide (215g) was added during 10 minutes to a solution containing ethyl 3-amino-4-chlorobenzoate (108g) and dimethyl sulphide (117ml) in dichloromethane maintaining below 0°C.
Triethylamine (80ml) was added and after 20 minutes further additions of dimethyl sulphide (117ml), N-chlorosuccinimide (215g) and triethylamine (80ml) were made. After 0.5 hours additional triethylamine (280ml) was introduced, the mixture heated under reflux conditions overnight and evaporated. Ether was added and this was washed with water, dilute sodium bicarbonate, dried (magnesium sulphate) and evaporated. Purification by chromatography, eluting with ethyl acetate/ petroleum ether gave ethyl 3-amino-4-chloro-2-methylsulphenylmethylbenzoate (47.6g), NMR (CDCl₃) 1.45(t,3H), 2.1(s,3H), 4.1(s,2H), 4.3(q,2H), 4.7(s,2H), 7.2(m,2H).

### REFERENCE EXAMPLE 15

n-Butyl lithium (2.5M in hexanes, 180ml) was added during 1 hour to a stirred solution of 3,4-dichlorobenzoic acid (40.0g) at -78°C and stirring maintained at that temperature overnight. A solution of methyl iodide (72ml) in tetrahydrofuran was then added during 1.5 hours and the mixture kept at -78°C for 3 hours and allowed to warm to room temperature overnight. The solvent was evaporated and the mixture added to water, acidified (concentrated hydrochloric acid) and extracted (ethyl acetate). The extract was dried (magnesium sulphate), evaporated and triturated (ether) to give 3,4-dichloro-2-methylbenzoic acid (33.3g) as a white solid, m.p. 177-178°C.

By proceeding in a similar manner the following compound was prepared:
3,4-difluoro-2-methylbenzoic acid, m.p. 152.5-153.5°C.

### REFERENCE EXAMPLE 16

A solution of sodium nitrite (13g) in concentrated sulphuric acid was added during 0.5 hours to a solution of ethyl 3-amino-4-chloro-2-methylbenzoate (22.4g) in acetic acid, keeping the temperature below 15°C. After stirring for a further 1 hour at 5°C, the resulting diazonium mixture was added during 0.75 hours to a solution of copper (I) bromide (31g) in hydrobromic acid (45%, 103ml) and water. Heating to 40°C was continued for a further 2 hours before addition of water and extraction (ethyl acetate). The extract was washed (sodium hydroxide solution), dried (magnesium sulphate) and evaporated to give ethyl 3-bromo-4-chloro-2-methylbenzoate (22.2g) as a brown oil, NMR (CDCl₃) 1.3(t,3H)), 2.65(s,3H), 4.3(q,2H), 7.45(m,2H).

Similarly prepared was ethyl 3-bromo-2-methylbenzoate, NMR (CDCl₃) 1.6(t,3H), 2.6(s,3H), 4.4(q,2H), 7.1(dd,1H), 7.65(d, 1H), 7.7(d,1H).

### REFERENCE EXAMPLE 17

A solution of ethyl 3-amino-4-chloro-2-(methylsulphenylmethyl)benzoate (50g) in ethanol was added to a stirred suspension of Raney Nickel (300g) in ethanol. After stirring overnight at room temperature, a further addition of Raney Nickel was made and the mixture stirred for 2 hours. Water was added, the mixture filtered and the residue washed with dichloromethane. The filtrate was evaporated, re-extracted with ethyl acetate, dried (magnesium sulphate) and evaporated to give ethyl 3-amino-4-chloro-2-methylbenzoate (29.7g) as a brown oil, NMR(CDCl₃) 1.25(t,3H), 2.25(s,3H), 4.3(q,2H), 7.05(m,2H).

### REFERENCE EXAMPLE 18

A mixture of 3,4-dichloro-2-methylbenzoic acid (33.3g) and thionyl chloride (200ml) was heated at reflux for 4 hours and then evaporated in vacuo. After re-evaporation of added toluene the residue was dissolved in ethanol and stirred overnight at ambient temperature. After evaporation the residue was dissolved in ether, washed with sodium bicarbonate solution, dried (magnesium sulphate) and evaporated to give ethyl 3,4-dichloro-2-methylbenzoate (34.2g), NMR(CDCl₃) 1.4(t,3H), 2.65(s,3H), 4.4(t,2H), 7.5(dd,2H).

By proceeding in a similar manner the following compound was prepared:
methyl 4-chloro-2-fluoro-3-methylbenzoate, NMR(CDCl₃) 2.43(s,3H), 3.95(s,3H), 7.21(m,1H), 7.71(m,1H).

### REFERENCE EXAMPLE 19

Sodium nitrite (28.6g) in sulphuric acid (150ml) was added dropwise to a solution of ethyl 3-amino-2-methylbenzoate (55.0g) in acetic acid, maintaining below 18°C. Stirring was continued for 1 hour at 15°C and the solution added dropwise to a mixture of copper (I) bromide (87.5g) and hydrobromic acid (300ml) and water (200ml) at 30°C. The resultant mixture was heated at 40°C for 4 hours, cooled, diluted with water and extracted (ethyl acetate). The extract was washed (water), dried (magnesium sulphate) and evaporated. Purification of the residue by chromatography on silica, eluting with ethyl acetate/petroleum ether (5:95) gave ethyl 3-bromo-2-methylbenzoate (52.4g).
NMR(CDCl₃) 1.6(t,3H), 2.6(s,3H), 4.4(q,2H), 7.1(dd,1H), 7.65(d,1H), 7.7(d,1H).

### REFERENCE EXAMPLE 20

A solution of n-butyllithium (189ml of a 2.5M solution in hexanes) was added to a stirred solution of 2-chloro-6-fluorotoluene (60.0g) in dry tetrahydrofuran (840ml) at -70°C. Stirring was maintained at this temperature overnight and the mixture poured onto excess solid CO₂. After warming to room temperature water was added and the mixture extracted into ether. The ether solution was extracted with water and the combined aqueous phases acidified with hydrochloric acid and extracted with ether. This extract was washed (water), dried (magnesium sulphate) and evaporated to give, after trituration with petroleum ether, 4-chloro-2-fluoro-3-methylbenzoic acid (61.5g) m.p. 194-195°C.

According to a feature of the present invention, there is provided a method for controlling the growth of weeds (i.e. undesired vegetation) at a locus which comprises applying to the locus a herbicidally effective amount of at least one 2-cyano-1,3-dione derivative of general formula (I) or an agriculturally acceptable salt thereof. For this purpose, the 2-cyano-1,3-dione derivatives are normally used in the form of herbicidal compositions (i.e. in association with compatible diluents or carriers and/or surface active agents suitable for use in herbicidal compositions), for example as hereinafter described.

The compounds of general formula (I) show herbicidal activity against dicotyledonous (i.e. broad-leafed) and monocotyledonous (e.g. grass) weeds by pre- and/or, post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of general formula (I) may be used to control the growth of
* broad-leafed weeds, for example, Abutilon theophrasti, Amaranthus retroflexus, Bidens pilosa, Chenopodium album, Galium aparine, Ipomoea spp. e.g. Ipomoea purpurea, Sesbania exaltata, Sinapis arvensis, Solanum nigrum and Xanthium strumarium, and
* grass weeds, for example Alopecurus myosuroides, Avena fatua, Digitaria sanguinalis, Echinochloa crus-galli, Eleusine indica and Setaria spp, e.g. Setaria faberii or Setaria viridis, and
* sedges, for example, Cyperus esculentus.

The amounts of compounds of general formula (I) applied vary with the nature of the weeds, the compositions used, the time of application, the climatic and edaphic conditions and (when used to control the growth of weeds in crop-growing areas) the nature of the crops. When applied to a crop-growing area, the rate of application should be sufficient to control the growth of weeds without causing substantial permanent damage to the crop. In general, taking these factors into account, application rates between 0.01 kg and 5 kg of active material per hectare give good results. However, it is to be understood that higher or lower application rates may be used, depending upon the particular problem of weed control encountered.

The compounds of general formula (I) may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for growing of crops, e.g. the crops hereinbefore mentioned, application rates between 0.01 kg and 4.0 kg, and preferably between 0.01 kg and 2 kg, of active material per hectare are particularly suitable.

The compounds of general formula (I) may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non- directional fashion (e.g. by directional or non-directional spraying) to the weeds or to the soil in which they are expected to appear, before or after planting of the trees or plantations at application rates between 0.25 kg and 5 kg, and preferably between 0.5 kg and 4 kg of active material per hectare.

The compounds of general formula (I) may also be used to control the growth of weeds, especially those indicated above, at loci which are not crop-growing areas but in which the control of weeds is nevertheless desirable.

Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise dosage will depend upon the nature of the vegetation treated and the effect sought.

Pre- or post-emergence application, and preferably pre-emergence application, in a directional or non-directional fashion (e.g. by directional or non-directional spraying) at application rates between 1 kg and 20 kg, and preferably between 5 and 10 kg, of active material per hectare are particularly suitable for this purpose.

When used to control the growth of weeds by pre-emergence application, the compounds of general formula (I) may be incorporated into the soil in which the weeds are expected to emerge. It will be appreciated that when the compounds of general formula (I) are used to control the growth of weeds by post-emergence application, i.e. by application to the aerial or exposed portions of emerged weeds, the compounds of general formula (I) will also normally come into contact with the soil and may also then exercise a pre-emergence control on later-germinating weeds in the soil.

Where especially prolonged weed control is required, the application of the compounds of general formula (I) may be repeated if required.

According to a further feature of the present invention, there are provided compositions suitable for herbicidal use comprising one or more of the 2-cyano-1,3-dione derivatives of general formula (I) or an agriculturally-acceptable salt thereof in association with, and preferably homogeneously dispersed in, one or more compatible herbicidally-acceptable diluents or carriers and/or surface active agents [i.e. diluents or carriers and/or surface active agents of the type generally accepted in the art as being suitable for use in herbicidal compositions and which are compatible with compounds of general formula (I)]. The term "homogeneously dispersed" is used to include compositions in which the compounds of general formula (I) are dissolved in other components. The term "herbicidal compositions" is used in a broad sense to include not only compositions which are ready for use as herbicides but also concentrates which must be diluted before use. Preferably, the compositions contain from 0.05 to 90% by weight of one or more compounds of general formula (I).

The herbicidal compositions may contain both a diluent or carrier and surface-active (e.g. wetting, dispersing, or emulsifying) agent. Surface-active agents which may be present in herbicidal compositions of the present invention may be of the ionic or non-ionic types, for example sulphoricinoleates, quaternary ammonium derivatives, products based on condensates of ethylene oxide with alkyl and polyaryl phenols, e.g. nonyl- or octyl-phenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl-and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the herbicidal compositions according to the present invention may comprise up to 10% by weight, e.g. from 0.05% to 10% by weight, of surface-active agent but, if desired, herbicidal compositions according to the present invention may comprise higher proportions of surface-active agent, for example up to 15% by weight in liquid emulsifiable suspension concentrates and up to 25% by weight in liquid water soluble concentrates.

Examples of suitable solid diluents or carriers are aluminium silicate, talc, calcined magnesia, kieselguhr, tricalcium phosphate, powdered cork, adsorbent carbon black and clays such as kaolin and bentonite. The solid compositions (which may take the form of dusts, granules or wettable powders) are preferably prepared by grinding the compounds of general formula (I) with solid diluents or by impregnating the solid diluents or carriers with solutions of the compounds of general formula (I) in volatile solvents, evaporating the solvents and, if necessary, grinding the products so as to obtain powders. Granular formulations may be prepared by absorbing the compounds of general formula (I) (dissolved in suitable solvents, which may, if desired, be volatile) onto the solid diluents or carriers in granular form and, if desired, evaporating the solvents, or by granulating compositions in powder form obtained as described above. Solid herbicidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use.

When desired, liquid compositions of the compound of general formula (I) may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Unless otherwise specified, the following percentages are by weight. Preferred herbicidal compositions according to the present invention are
* aqueous suspension concentrates which comprise from 10 to 70% of one or more compounds of general formula (I), from 2 to 10% of surface-active agent, from 0.1 to 5% of thickener and from 15 to 87.9% of water,
* wettable powders which comprise from 10 to 90% of one or more compounds of general formula (I), from 2 to 10% of surface-active agent and from 8 to 88% of solid diluent or carrier,
* soluble powders which comprise from 10 to 90% of one or more compounds of general formula (I), from 2 to 40% of sodium carbonate and from 0 to 88% of solid diluent
* liquid water soluble concentrates which comprise from 5 to 50%, e.g. 10 to 30%, of one or more compounds of general formula (I), from 5 to 25% of surface-active agent and from 25 to 90%, e.g. 45 to 85%, of water miscible solvent, e.g. dimethylformamide, or a mixture of water-miscible solvent and water,
* liquid emulsifiable suspension concentrates which comprise from 10 to 70% of one or more compounds of general formula (I), from 5 to 15% of surface-active agent, from 0.1 to 5% of thickener and from 10 to 84.9% of organic solvent
* granules which comprise from 1 to 90%, e.g. 2 to 10% of one or more compounds of general formula (I), from 0.5 to 7%, e.g. 0.5 to 2%, of surface-active agent and from 3 to 98.5%, e.g. 88 to 97. 5%, of granular carrier and,
* emulsifiable concentrates which comprise 0.05 to 90%, and preferably from 1 to 60% of one or more compounds of general formula (I), from 0.01 to 10%, and preferably from 1 to 10%, of surface-active agent and from 9.99 to 99.94%, and preferably from 39 to 98.99%, of organic solvent.

Herbicidal compositions according to the present invention may also comprise the compounds of general formula (I) in association with, and preferably homogeneously dispersed in, one or more other pesticidally active compounds and, if desired, one or more compatible pesticidally acceptable diluents or carriers, surface-active agents and conventional adjuvants as hereinbefore described. Examples of other pesticidally active compounds which may be included in, or used in conjunction with, the herbicidal compositions of the present invention include herbicides, for example to increase the range of weed species controlled for example alachlor [2-chloro-2,6'-diethyl-N-(methoxy-methyl)-acetanilide], atrazine [2-chloro-4-ethylarnino-6-isopropylamino-1,3,5-triazine], bromoxynil [3,5-dibromo-4-hydroxybenzonitrile], chlortoluron [N'-(3-chloro-4-methylphenyl)-N,N-dimethylurea], cyanazine [2-chloro-4-(1-cyano-1-methylethylamino)-6-ethylamino-1,3,5-triazine], 2,4-D [2,4-dichlorophenoxy-acetic acid], dicamba [3,6-dichloro-2-methoxybenzoic acid], difenzoquat [1,2-dimethyl-3,5-diphenyl-pyrazolium salts], flampropmethyl [methyl N-2-(N- benzoyl-3-chloro-4-fluoroanilino)-propionate], fluometuron [N'-(3-trifluoro-methylphenyl)-N,N-dimethylurea], isoproturon [N'-(4-isopropylphenyl)-N,N-dimethylurea], nicosulfuron [2-(4,6-dimethoxypyrimidin-2-ylcarbamoylsulfamoyl)-N,N-dimethylnicotinamide] insecticides, e.g. synthetic pyrethroids, e.g. permethrin and cypermethrin, and fungicides, e.g. carbamates, e.g. methyl N-(1-butyl-carbamoylbenzimidazol-2-yl)carbamate, and triazoles e.g. 1-(4-chloro-phenoxy)-3,3-dimethyl-1(1,2,4-triazol-1-yl)-butan-2-one.

Pesticidally active compounds and other biologically active materials which may be included in, or used in conjunction with, the herbicidal compositions of the present invention, for example those hereinbefore mentioned, and which are acids, may, if desired, be utilized in the form of conventional derivatives, for example alkali metal and amine salts and esters.

According to a further feature of the present invention there is provided an article of manufacture comprising at least one of the 2-cyano-1,3-dione derivatives of general formula (I) or an agriculturally-acceptable salt thereof or, as is preferred, a herbicidal composition as hereinbefore described, and preferably a herbicidal concentrate which must be diluted before use, comprising at least one of the 2-cyano-1,3-dione derivatives of general formula (I) within a container for the aforesaid derivative or derivatives of general formula (I), or a said herbicidal composition, and instructions physically associated with the aforesaid container setting out the manner in which the aforesaid derivative or derivatives of general formula (I) or herbicidal composition contained therein is to be used to control the growth of weeds. The containers will normally be of the types conventionally used for the storage of chemical substances which are solid at normal ambient temperatures and herbicidal compositions particularly in the form of concentrates, for example cans and drums of metal, which may be internally lacquered, and plastics materials, bottles or glass and plastics materials and, when the contents of the container is a solid, for example granular, herbicidal compositions, boxes, for example of cardboard, plastics materials and metal, or sacks. The containers will normally be of sufficient capacity to contain amounts of the 2-cyano-1,3-dione derivative or herbicidal compositions sufficient to treat at least one acre of ground to control the growth of weeds therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to control the growth of weeds at rates of application between 0.01 kg and 20 kg of active material per hectare in the manner and for the purposes hereinbefore described.

The following Examples illustrate herbicidal compositions according to the present invention:

### EXAMPLE C1

A wettable powder is formed from:
* active ingredient (compound 1): 50% w/w
* nonylphenol/ethylene oxide condensate containing 9 moles of ethylene oxide per mol of phenol: 5% w/w
* silicon dioxide of micro-fine particle size: 5% w/w
* synthetic magnesium silicate carrier: 40% w/w

by absorbing the condensate on the silicon dioxide, mixing with the other ingredients and grinding the mixture in a hammermill to give a wettable powder.

Similar wettable powders may be prepared as described above by replacing the 2-cyano-1,3-dione (compound 1) by other compounds of formula (I).

### EXAMPLE C2

An aqueous suspension concentrate is formed from:
* active ingredient (compound 1): 50% w/v
* nonylphenol/ethylene oxide condensate containing 9 moles of ethylene oxide per mol of phenol: 1 % w/v
* sodium salt of polycarboxylic acid: 0.2% w/v
* Ethylene glycol : 5% w/v
* polysaccaride xanthan gum thickener: 0.15% w/v
* water to 100% by volume
by intimately mixing the ingredients and grinding in a ball-mill for 24 hours.

Similar aqueous concentrates may be prepared as described above by replacing the 2-cyano-1,3-dione (compound 1) by other compounds of general formula (I).

Representative compounds of formula (I) have been used in herbicidal applications according to the following procedures.

### Method of use of herbicidal compounds:

### Herbicidal activity:

Appropriate quantities of the compounds used to treat the plant were dissolved in acetone to give solutions equivalent to an application rate of up to 1000g of the compounds used to treat the plants per hectare (g/ha). These solutions were applied at 260 litres of spray fluid per hectare.

### a) Pre-emergence application weed control.

Seeds (weeds or crops) were sown in loam soil pots.

The compounds of the invention were applied to the soil surface as described above.

### b) Post-emergence application weed control.

Weed species were grown until ready for spraying with the compounds of the invention . The growth stage of the plants at spraying were as follows :

| 1) Broad-leafed weeds | |
|---|---|
| Abutilon theophrasti | 1-2 leaves. |
| Amaranthus retroflexus | 1-2 leaves. |
| Galium aparine | 1-2 whorls. |
| Sinapis arvensis | 2 leaves. |
| Ipomoea purpurea | 1-2 leaves. |
| Xanthium strumarium | 2 leaves. |

| 2) Grass weeds : | |
|---|---|
| Alopecurus myosuroides: | 2 leaves. |
| Avena fatua : | 1-2 leaves. |
| Echinochloa crus-galli | 2-3 leaves. |
| Setaria viridis : | 2-3 leaves. |

| 3) Sedges | |
|---|---|
| Cyperus esculentus | 3 leaves. |

### c) Crop tolerance

Compounds of the invention were applied pre-emergence as in (a) or post emergence (3-leaf stage) to the following crops:- wheat, maize, rice, soya and cotton.

A single pot of each plant species was allocated to each treatment with unsprayed controls and controls sprayed with acetone alone.

After treatment, the pots were kept in the greenhouse and were watered overhead.

Visual assessment of phytotoxicity was made 17-20 days after spraying. Weed control results were expressed as the percentage reduction in growth or kill of the weeds, in comparison with the plants in the control pots. Crop tolerance was expressed as the percentage damage to crop.

Representative compounds of the invention, when used at an application rate of 1 kg/ha or less, show herbicidal activity against one or more of the weed species listed above: such compounds also show selectivity on one or more of the listed crop species.

## Claims

1. A 2-cyano-1,3-dione derivative of the formula: 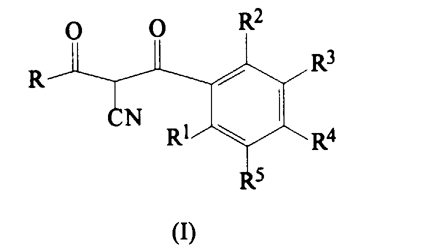 wherein:
R represents:-
a straight- or branched- chain alkyl group containing up to six carbon atoms which is optionally substituted by one or more halogen atoms; or
a cycloalkyl group containing from three to six carbon atoms optionally substituted by one or more R⁷ groups;
R¹ represents the hydrogen atom;
R², R³, R⁴ and R⁵, which may be the same or different, each represents -
the hydrogen atom;
a halogen atom;
a straight- or branched- chain alkyl, alkenyl or alkynyl group containing up to six carbon atoms optionally substituted by one or more halogen atoms;
phenyl optionally substituted by up to three groups R²¹ which may be the same or different;
a straight- or branched- chain alkyl group containing up to six carbon atoms which is substituted by a group -OR⁶;
a group selected from nitro, cyano, -CO₂R⁶,-COR⁷, -S(O)ₙR⁹, -O(CH₂)ₘOR⁶, -N(R¹²)SO₂R⁸, -OR⁶¹, -OSO₂R⁸, -CONR¹⁰R¹⁵ and -(CR¹³R¹⁴)ₜ-S(O)_{q}R⁸;
provided that at least one of the groups R² to R⁵ represents -(CR¹³R¹⁴)ₜ-S(O)_{q}R⁸;
R⁶ represents a straight- or branched- chain alkyl, alkenyl or alkynyl group containing up to six carbon atoms optionally substituted by one or more halogen atoms; or
a cycloalkyl group containing from three to six carbon atoms;
R⁶¹ represents:-
a straight- or branched- chain alkyl, alkenyl or alkynyl group containing up to six carbon atoms optionally substituted by one or more halogen atoms;
a cycloalkyl group containing from three to six carbon atoms;
or phenyl optionally substituted by from one to five groups R²¹ which may be the same or different;
R⁷ represents a straight- or branched- chain alkyl group containing up to six carbon atoms optionally substituted by one or more halogen atoms; or
a cycloalkyl group containing from three to six carbon atoms;
R⁸ represents:-
a straight- or branched- chain alkyl, alkenyl or alkynyl group containing up to six carbon atoms optionally substituted by one or more halogen atoms;
a cycloalkyl group containing from three to six carbon atoms;
phenyl optionally substituted by from one to five groups R²¹ which may be the same or different; or
-NR¹⁰R¹¹;
R⁹ represents:-
a straight- or branched- chain alkyl, alkenyl or alkynyl group containing up to six carbon atoms optionally substituted by one or more halogen atoms;
a cycloalkyl group containing from three to six carbon atoms;
or phenyl optionally substituted by from one to five groups R²¹ which may be the same or different;
R¹⁰ represents hydrogen a straight- or branched- chain alkyl group containing up to six carbon atoms optionally substituted by one or more halogen atoms;
R¹¹ represents:-
a straight- or branched- chain alkyl group containing up to six carbon atoms optionally substituted by one or more halogen atoms;
or a group selected from -COR⁷, -CO₂R⁷ and -CONR⁷R¹⁰;
where R¹⁰ and R¹¹ are part of a group -NR¹⁰R¹¹ they may, together with the nitrogen to which they are attached, may form a 5 or 6 membered ring optionally having one additional heteroatom in the ring which is oxygen or nitrogen, wherein the ring is optionally substituted by one or more alkyl groups containing up to three carbon atoms;
R¹² represents:
the hydrogen atom;
a straight- or branched- chain alkyl, alkenyl or alkynyl group containing up to six carbon atoms optionally substituted by one or more halogen atoms;
a cycloalkyl group containing from three to six carbon atoms; phenyl optionally substituted by from one to five groups R²¹ which may be the same or different;
or a group -OR¹⁷;
R¹³ and R¹⁴, which may be the same or different, each represents the hydrogen atom or a straight- or branched- chain alkyl group containing up to six carbon atoms optionally substituted by one or more halogen atoms;
R¹⁵ represents a group selected from R⁷ and -OR¹⁷;
where R¹⁰ and R¹⁵ are part of a group -CONR¹⁰R¹⁵ they may, together with the nitrogen to which they are attached, form a five or six membered ring optionally having one additional heteroatom in the ring which is oxygen or nitrogen, wherein the ring is optionally substituted by one or more alkyl groups containing up to three carbon atoms;
R¹⁷ represents a straight- or branched- chain alkyl group containing up to six carbon atoms;
R²¹ represents
a halogen atom;
a straight- or branched- chain alkyl group containing up to three carbon atoms optionally substituted by one or more halogen atoms;
or a group selected from nitro, cyano, -S(O)ₙR⁷ and -OR⁷;
m represents one, two or three;
n represents zero, one or two;
q represents zero, one or two; and
t represents an integer from one to four; where t is greater than one the groups -(CR¹³R¹⁴)- may be the same or different.

2. A compound according to claim 1 wherein:
R represents:-
a straight- or branched- chain alkyl group containing up to six carbon atoms optionally substituted by one or more halogen atoms; or
a cycloalkyl group containing from three to six carbon atoms optionally substituted by one or more methyl groups;
R², R³, R⁴ and R⁵, which may be the same or different, each represents:-
a hydrogen or halogen atom; or
a straight- or branched- chain alkyl, alkenyl or alkynyl group containing up to six carbon atoms optionally substituted by one or more halogen atoms;
a straight- or branched- chain alkyl group containing up to six carbon atoms which is substituted by -OR⁶; or
a group selected from -COR⁷, -CO₂R⁶, cyano, nitro, -O(CH₂)ₘOR⁶; -OR⁶¹, -N(R¹²)SO₂R⁸, -OSO₂R⁸, -S(O)ₙR⁹ and -(CR¹³R¹⁴)ₜSO_{q}R⁸;
R⁶ and R⁷, which may be the same or different each represents:-
a straight- or branched- chain alkyl group containing up to six carbon atoms optionally substituted by one or more halogen atoms;
or a cycloalkyl group containing three or four carbon atoms;
R⁶¹ and R⁹, which may be the same or different, each represents:-
a straight- or branched- chain alkyl or alkenyl group containing up to six carbon atoms optionally substituted by one or more halogen atoms;
a straight- or branched- alkynyl group containing from three to six carbon atoms optionally substituted by one or more halogen atoms;
a cycloalkyl group containing three to six carbon atoms;
R⁸ represents:-
a straight- or branched- chain alkyl or alkenyl group containing up to six carbon atoms optionally substituted by one or more halogen atoms;
a straight- or branched- alkynyl group containing from three to six carbon atoms optionally substituted by one or more halogen atoms;
a cycloalkyl group containing three to six carbon atoms; or
phenyl optionally substituted by from one to three groups R²¹ which may be the same or different;
R¹² represents:-
the hydrogen atom;
a straight- or branched- chain alkyl or alkenyl group containing up to six carbon atoms optionally substituted by one or more halogen atoms;
a straight- or branched- alkynyl group containing from three to six carbon atoms optionally substituted by one or more halogen atoms;
a cycloalkyl group containing three to six carbon atoms;
R¹³ and R¹⁴, which may be the same or different, each represents:-
a hydrogen atom; or
a straight- or branched- chain alkyl group containing up to six carbon atoms optionally substituted by one or more halogen atoms;
R²¹ represents:-
a halogen atom;
a straight- or branched- chain alkyl group containing up to three carbon atoms optionally substituted by one or more halogen atoms; or
a group selected from nitro, cyano, -S(O)ₙR⁷ and -OR⁷;
m represents two or three;
n represents zero, one or two;
q represents zero, one or two; and
t represents one or two.

3. A compound according to claim 1 wherein:
R represents:-
a straight- or branched chain alkyl group containing up to three carbon atoms; or
a cycloalkyl group containing thee or four carbon atoms optionally substituted by one or more methyl groups;
R^{2,} R³ and R⁴, which may be the same or different, each represents:-
a hydrogen, chlorine, bromine or fluorine atom; or
a straight- or branched- chain alkyl or alkenyl group containing up to four carbon atoms optionally substituted by one or more chlorine, bromine or fluorine atoms;
a straight- or branched- chain alkynyl group containing up to four carbon atoms;
a straight- or branched- chain alkyl group containing up to four carbon atoms which is substituted by -OR⁶; or
a group selected from -COR⁷, -CO₂R⁶, -S(O)ₙR⁹, -O(CH₂)ₘOR⁶, -N(R¹²)SO₂R⁸, -OR⁶¹, -(CR¹³R¹⁴)ₜS(O)_{q}R⁸ and -OSO₂R⁸;
provided at least one of the groups R² to R⁴ represents -(CR¹³R¹⁴)ₜ-S(O)_{q}R⁸;
R⁵ represents the hydrogen atom;
R⁶, R⁷, R⁸ and R⁹, which may be the same or different, each represents:-
a straight- or branched- chain alkyl group containing up to four carbon atoms optionally substituted by one or more chlorine, bromine or fluorine atoms; or
a cyclopropyl group;
R⁶¹ represents:-
a straight- or branched- chain alkyl or alkenyl group containing up to four carbon atoms optionally substituted by one or more chlorine, bromine or fluorine atoms;
a straight- or branched- chain alkynyl group containing three or four carbon atoms, or
a cyclopropyl group;
R¹² represents:-
the hydrogen atom; or
a straight- or branched- chain alkyl or alkenyl group containing up to four carbon atoms optionally substituted by one or more chlorine, bromine or fluorine atoms;
a straight- or branched- chain alkynyl group containing three or four carbon atoms, or
a cyclopropyl group;
R¹³ and R¹⁴ which may be the same or different, each represents:-
the hydrogen atom; or
a straight- or branched- chain alkyl group containing up to three carbon atoms;
m represents two or three;
n represents zero, one or two;
q represents zero, one or two; and
t represents one.

4. A compound according to claim 1 wherein:
R represents:-
a methyl, ethyl, isopropyl, cyclopropyl or 1-methylcyclopropyl group;
R², R³ and R⁴, which may be the same or different, each represents:-
a hydrogen, chlorine, bromine or fluorine atom; or
a straight- or branched- chain alkyl or alkenyl group containing up to four carbon atoms optionally substituted by one or more chlorine, bromine or fluorine atoms;
a straight- or branched- chain alkyl group containing up to three carbon atoms which is substituted by -OR⁶; or
a group selected from -COR⁷, -CO₂R⁶, -SR⁹, -O(CH₂)ₘOR⁶, -OR⁶¹, -N(R¹²)SO₂R⁸, -OSO₂R⁸ and -(CR¹³R¹⁴)ₜS(O)_{q}R⁸;
provided at least one of the groups R² to R⁴ represents -(CR¹³R¹⁴)ₜ-S(O)_{q}R⁸;
R⁵ represents the hydrogen atom;
R⁶, R⁷, R⁸ and R⁹, which may be the same or different, each represents:-
a straight- or branched- chain alkyl group containing up to three carbon atoms;
R⁶¹ represents:-
a straight- or branched- chain alkyl group containing up to four carbon atoms optionally substituted by one or more chlorine, bromine or fluorine atoms;
a straight- or branched- chain alkenyl or alkynyl group containing three or four carbon atoms; or
a cyclopropyl group;
R¹² represents:-
the hydrogen atom; or
a straight- or branched- chain alkyl group containing up to three carbon atoms optionally substituted by one or more chlorine, bromine or fluorine atoms;
an allyl group optionally substituted by one or more chlorine, bromine or fluorine atoms;
R¹³ and R¹⁴, which may be the same or different, each represents the hydrogen atom, or a methyl or ethyl group;
m represents two or three;
q represents zero, one or two; and
t represents one.

5. A compound according to claim 1 wherein:
(a) R is cyclopropyl, R¹ is hydrogen, R² is -CH₂SO₂CH₃, R³ is hydrogen, R⁴ is bromo and R⁵ is hydrogen;
(b) R is cyclopropyl, R¹ is hydrogen, R² is chloro, R³ is hydrogen, R⁴ is -CH₂SCH₃ and R⁵ is hydrogen;
(c) R is cyclopropyl, R¹ is hydrogen, R² is -CH₂SCH₃, R³ is bromine, R⁴ is chlorine and R⁵ is hydrogen;
(d) R is cyclopropyl, R¹ is hydrogen, R² is -CH₂SO₂CH₂CH₃, R³ and R⁴ are chlorine and R⁵ is hydrogen;
(e) R is cyclopropyl, R¹ is hydrogen, R² is -CH₂SCH₃, R³ is bromine and R⁴ and R⁵ are hydrogen;
(f) R is cyclopropyl, R¹ is hydrogen, R² is -CH₂SO₂CH₃ and R³, R⁴ and R⁵ are hydrogen;
(g) R is cyclopropyl, R¹ is hydrogen, R² is -CH₂SCH₃, R³ and R⁴ are fluorine and R⁵ is hydrogen;
(h) R is cyclopropyl, R¹ is hydrogen, R² is chlorine, R³ is hydrogen, R⁴ is -CH₂SCH₃ and R⁵ is hydrogen;
(i) R is cyclopropyl, R¹ is hydrogen, R² is -SCH₃, R³ is -CH₂SCH(CH₃)CH₃, R⁴ is chlorine and R⁵ is hydrogen;
or an agriculturally acceptable salt, metal complex or enolic tautomeric form thereof.

6. A herbicidal composition comprising a herbicidally effective amount of a 2-cyano-1,3-dione derivative of formula (I) according to any one of claims 1 to 5, or an agriculturally acceptable salt, metal complex or enolic tautomeric form thereof, in association with an agriculturally acceptable diluent or carrier and/or surface active agent.

7. A herbicidal composition according to claim 6 in the form of an aqueous suspension concentrate, a wettable powder, a water soluble or water dispersible powder, a liquid water soluble concentrate, a liquid emulsifiable suspension concentrate, a granule or an emulsifiable concentrate.

8. A method for controlling the growth of weeds at a locus which comprises applying to the locus a herbicidally effective amount of a 2-cyano-1,3-dione derivative of formula (I) as defined in any one of claims 1 to 5 or an agriculturally acceptable salt, metal complex or enolic tautomeric form thereof.

9. A method according to claim 8 in which the locus is an area used, or to be used, for growing of crops and the compound is applied at an application rate from 0.01 kg to 4.0 kg per hectare.

10. A process for the preparation of a 2-cyano-1,3-dione derivative of formula (I) as defined in claim 1 which comprises:
(a) reacting a compound of formula (II): 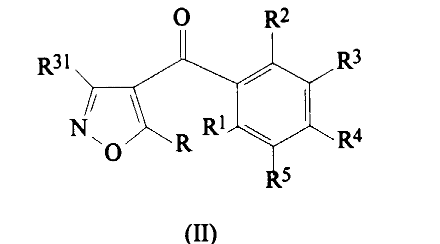 wherein R, R¹, R^{2,} R³, R⁴ and R⁵ are as defined in claim 1 and R³¹ represents the hydrogen atom or an acyl group, with a base; or
(b) subjecting a compound of formula (II) above wherein R³¹ represents an ester, amide or nitrile, to acidic or basic hydrolysis; or
(c) reacting a compound of formula (III): 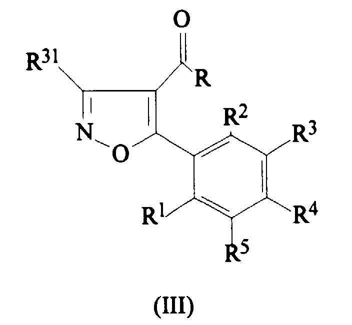 wherein R, R¹, R², R³, R⁴ and R⁵ are as defined in claim 1 and R³¹ is hydrogen or an acyl group, with a base; or
(d) subjecting a compound of formula (III) above wherein R³¹ represents an ester, amide or nitrile, to acidic or basic hydrolysis; or
(e) reacting a benzoyl chloride of formula (IV): 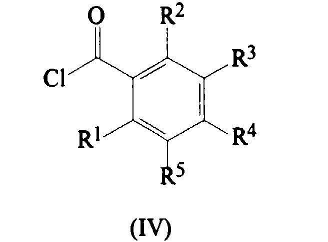 wherein R¹ R^{2,} R³, R⁴ and R⁵ are as defined in claim 1, with a beta-ketonitrile of formula(V): 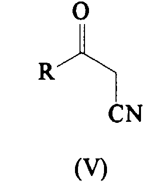 wherein R is as defined in claim 1; or
(f) reacting an acid chloride of formula (VI): 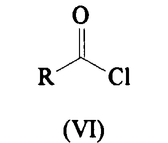 wherein R is as defined in claim 1, with a beta-ketonitrile of formula (VII): 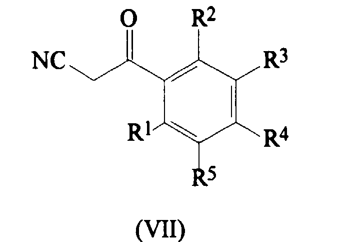 wherein R¹, R², R³, R⁴ and R⁵ are as defined in claim 1; or
(g) reacting a benzoyl chloride of formula (IV) above with a beta-ketonitrile of formula (V) in the presence of a mild base to form an intermediate of formula (VIII): 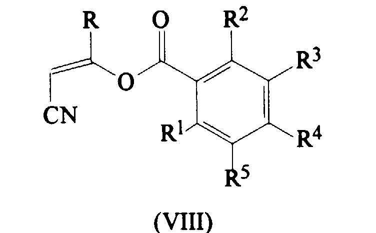 wherein R, R¹, R², R³, R⁴ and R⁵ are as defined in claim 1, followed by effecting rearrangement of the intermediate of formula (VIII) in the presence of a catalyst; or
(h) reacting an acid chloride of formula (VI) as defined above with a beta-ketonitrile of formula (VII) as defined above in the presence of a mild base to form an intermediate of formula (IX): 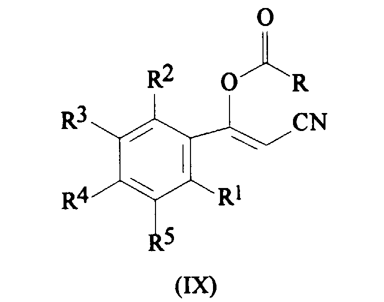 wherein R, R¹, R², R³, R⁴ and R⁵ are as defined in claim 1, followed by effecting rearrangement of the intermediate of formula (IX) in the presence of a catalyst;
optionally followed by the conversion of the compound thus obtained into an agriculturally acceptable salt or metal complex thereof.

## Patentansprüche

1. 2-Cyano-1,3-dion-Derivat der Formel: 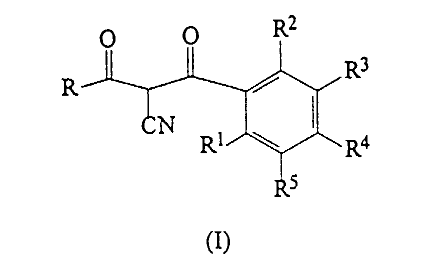 in der bedeuten:
R eine geradkettige oder verzweigte Alkylgruppe, die bis zu 6 Kohlenstoffatome enthält, welche gegebenenfalls mit einem oder mehreren Halogenatomen substituiert ist, oder
eine Cycloalkylgruppe, die 3 bis 6 Kohlenstoffatome enthält, welche gegebenenfalls mit einer oder mehreren Gruppen R⁷ substituiert ist,
R¹ das Wasserstoffatom,
R^{2,} R³, R⁴ und R⁵, die gleich oder voneinander verschieden sein können, jeweils:
das Wasserstoffatom,
ein Halogenatom,
eine geradkettige oder verzweigte Alkyl-, Alkenyl- oder Alkinylgruppe, die bis zu 6 Kohlenstoffatome enthält, welche gegebenenfalls mit einem oder mehreren Halogenatomen substituiert ist,
Phenyl, das gegebenenfalls mit bis zu drei Gruppen R²¹, die gleich oder voneinander verschieden sein können, substituiert ist,
eine geradkettige oder verzweigte Alkylgruppe, die bis zu 6 Kohlenstoffatome enthält, welche durch eine Gruppe -OR⁶ substituiert ist,
eine unter Nitro, Cyano, -CO₂R⁶, -COR⁷, -S(O)ₙR⁹, -O(CH₂)ₘOR⁶, -N(R¹²)SO₂R⁸, -OR⁶¹, -OSO₂R⁸, -CONR¹⁰R¹⁵ und - (CR¹³R¹⁴)ₜ-S(O)_{q}R⁸ ausgewählte Gruppe,
mit der Maßgabe, daß mindestens eine der Gruppen R² bis R⁵ =(CR¹³R¹⁴)ₜ-S(O)_{q}R⁸ bedeutet,
R⁶ eine geradkettige oder verzweigte Alkyl-, Alkenyl- oder Alkinylgruppe, die bis zu 6 Kohlenstoffatome enthält, welche gegebenenfalls mit einem oder mehreren Halogenatomen substituiert ist, oder
eine Cycloalkylgruppe, die 3 bis 6 Kohlenstoffatome enthält,
R⁶¹ eine geradkettige oder verzweigte Alkyl-, Alkenyl- oder Alkinylgruppe, die bis zu 6 Kohlenstoffatome enthält, welche gegebenenfalls mit einem oder mehreren Halogenatomen substituiert ist,
eine Cycloalkylgruppe, die 3 bis 6 Kohlenstoffatome enthält, oder
Phenyl, das gegebenenfalls mit 1 bis 5 Gruppen R²¹, die gleich oder voneinander verschieden sein können, substituiert ist,
R⁷ eine geradkettige oder verzweigte Alkylgruppe, die bis zu 6 Kohlenstoffatome enthält, welche gegebenenfalls mit einem oder mehreren Halogenatomen substituiert ist, oder
eine Cycloalkylgruppe, die 3 bis 6 Kohlenstoffatome enthält,
R⁸ eine geradkettige oder verzweigte Alkyl-, Alkenyl- oder Alkinylgruppe, die bis zu 6 Kohlenstoffatome enthält, welche gegebenenfalls mit einem oder mehreren Halogenatomen substituiert ist,
eine Cycloalkylgruppe, die 3 bis 6 Kohlenstoffatome enthält,
Phenyl, das gegebenenfalls mit 1 bis 5 Gruppen R²¹, die gleich oder voneinander verschieden sein können, substituiert ist, oder
-NR¹⁰R¹¹,
R⁹ eine geradkettige oder verzweigte Alkyl-, Alkenyl- oder Alkinylgruppe, die bis zu 6 Kohlenstoffatome enthält, welche gegebenenfalls mit einem oder mehreren Halogenatomen substituiert ist,
eine Cycloalkylgruppe, die 3 bis 6 Kohlenstoffatome enthält,
oder Phenyl, das gegebenenfalls mit 1 bis 5 Gruppen R²¹, die gleich oder voneinander verschieden sein können, substituiert ist,
R¹⁰ Wasserstoff, eine geradkettige oder verzweigte Alkylgruppe, die bis zu 6 Kohlenstoffatomen enthält, welche gegebenenfalls mit einem oder mehreren Halogenatomen substituiert ist,
R¹¹ eine geradkettige oder verzweigte Alkylgruppe, die bis zu 6 Kohlenstoffatome enthält, die gegebenenfalls mit einem oder mehreren Halogenatomen substituiert ist, oder
eine Gruppe, die unter -COR⁷, -CO₂R⁷ und -CONR⁷R¹⁰ ausgewählt ist,
wobei, wenn R¹⁰ und R¹¹ Teile einer Gruppe -NR¹⁰R¹¹ sind, sie zusammen mit dem Stickstoff, an den sie gebunden sind, einen 5- oder 6-gliedrigen Ring bilden können, der gegebenenfalls ein zusätzliches Heteroatom im Ring, nämlich Sauerstoff oder Stickstoff, aufweist, wobei der Ring gegebenenfalls mit einer oder mehreren Alkylgruppen, die bis zu 3 Kohlenstoffatome enthalten, substituiert ist,
R¹² das Wasserstoffatom,
eine geradkettige oder verzweigte Alkyl-, Alkenyl- oder Alkinylgruppe, die bis zu 6 Kohlenstoffatome enthält, welche gegebenenfalls mit einem oder mehreren Halogenatomen substituiert ist,
eine Cycloalkylgruppe, die 3 bis 6 Kohlenstoffatome enthält,
Phenyl, das gegebenenfalls mit 1 bis 5 Gruppen R²¹, die gleich oder voneinander verschieden sein können, substituiert ist,
oder eine Gruppe -OR¹⁷,
R¹³ und R¹⁴, die gleich oder voneinander verschieden sein können, jeweils das Wasserstoffatom oder eine geradkettige oder verzweigte Alkylgruppe, die bis zu 6 Kohlenstoffatome enthält, welche gegebenenfalls mit einem oder mehreren Halogenatomen substituiert ist,
R¹⁵ eine unter R⁷ und -OR¹⁷ ausgewählte Gruppe,
wobei, wenn R¹⁰ und R¹⁵ Teil einer Gruppe -CONR¹⁰R¹⁵ sind, sie zusammen mit dem Stickstoff, an den sie gebunden sind, einen 5- oder 6-gliedrigen Ring bilden können, der gegebenenfalls ein zusätzliches Heteroatom im Ring, nämlich Sauerstoff oder Stickstoff, aufweist, wobei der Ring gegebenenfalls mit einer oder mehreren Alkylgruppen, die bis zu drei Kohlenstoffatome enthalten, substituiert ist,
R¹⁷ eine geradkettige oder verzweigte Alkylgruppe, die bis zu 6 Kohlenstoffatome enthält,
R²¹ ein Halogenatom,
eine geradkettige oder verzweigte Alkylgruppe, die bis zu 3 Kohlenstoffatome enthält, welche gegebenenfalls mit einem oder mehreren Halogenatomen substituiert ist,
oder eine Gruppe, die unter Nitro, Cyano, -S(O)ₙR⁷ und -OR⁷ ausgewählt ist,
m 1, 2 oder 3,
n 0, 1 oder 2,
q 0, 1 oder 2, und
t eine ganze Zahl von 1 bis 4, wobei, wenn t größer als 1 ist, die Gruppen -(CR¹³R¹⁴)- gleich oder voneinander verschieden sein können.

2. Verbindung nach Anspruch 1, wobei bedeuten:
R eine geradkettige oder verzweigte Alkylgruppe, die bis zu 6 Kohlenstoffatome enthält, welche gegebenenfalls mit einem oder mehreren Halogenatomen substituiert ist, oder
eine Cycloalkylgruppe, die 3 bis 6 Kohlenstoffatome enthält, welche gegebenenfalls mit einer oder mehreren Methylgruppen substituiert ist,
R^{2,} R³, R⁴ und R⁵, die gleich oder voneinander verschieden sein können, jeweils
ein Wasserstoff- oder Halogenatom, oder
eine geradkettige oder verzweigte Alkyl-, Alkenyl- oder Alkinylgruppe, die bis zu 6 Kohlenstoffatome enthält, welche gegebenenfalls mit einem oder mehreren Halogenatomen substituiert ist,
eine geradkettige oder verzweigte Alkylgruppe, die bis zu 6 Kohlenstoffatome enthält, welche durch -OR⁶ substituiert ist, oder
eine Gruppe, die unter -COR⁷, -CO₂R⁶, Cyano, Nitro, -O(CH₂)ₘOR⁶, -OR⁶¹, -N(R¹²)SO₂R⁸, -OSO₂R⁸, -S(O)ₙR⁹ und -(CR¹³R¹⁴)ₜSO_{q}R⁸ ausgewählt ist,
R⁶ und R⁷, die gleich oder voneinander verschieden sein können, jeweils eine geradkettige oder verzweigte Alkylgruppe, die bis zu 6 Kohlenstoffatome enthält, welche gegebenenfalls mit einem oder mehreren Halogenatomen substituiert ist, oder
eine Cycloalkylgruppe, die 3 bis 4 Kohlenstoffatome enthält,
R⁶¹ und R⁹, die gleich oder voneinander verschieden sein können, jeweils eine geradkettige oder verzweigte Alkyl- oder Alkenylgruppe, die bis zu 6 Kohlenstoffatome enthält, welche gegebenenfalls mit einem oder mehreren Halogenatomen substituiert ist,
eine geradkettige oder verzweigte Alkinylgruppe, die 3 bis 6 Kohlenstoffatome enthält, welche gegebenenfalls mit einem oder mehreren Halogenatomen substituiert ist,
eine Cycloalkylgruppe, die 3 bis 6 Kohlenstoffatome enthält,
R⁸ eine geradkettige oder verzweigte Alkyl- oder Alkenylgruppe, die bis zu 6 Kohlenstoffatom enthält, welche gegebenenfalls mit einem oder mehreren Halogenatomen substituiert ist,
eine geradkettige oder verzweigte Alkinylgruppe, die 3 bis 6 Kohlenstoffatome enthält, welche gegebenenfalls mit einem oder mehreren Halogenatomen substituiert ist,
eine Cycloalkylgruppe, die 3 bis 6 Kohlenstoffatome enthält, oder
Phenyl, das gegebenenfalls mit 1 bis 3 Gruppen R²¹, die gleich oder voneinander verschieden sein können, substituiert ist,
R¹² das Wasserstoffatom,
eine geradkettige oder verzweigte Alkyl- oder Alkenylgruppe, die bis zu 6 Kohlenstoffatome enthält, welche gegebenenfalls mit einem oder mehreren Halogenatomen substituiert ist,
eine geradkettige oder verzweigte Alkinylgruppe, die 3 bis 6 Kohlenstoffatome enthält, welche gegebenenfalls mit einem oder mehreren Halogenatomen substituiert ist,
eine Cycloalkylgruppe, die 3 bis 6 Kohlenstoffatome enthält,
R¹³ und R¹⁴, die gleich oder voneinander verschieden sein können, jeweils
ein Wasserstoffatom oder
eine geradkettige oder verzweigte Alkylgruppe, die bis zu 6 Kohlenstoffatome enthält, welche gegebenenfalls mit einem oder mehreren Halogenatomen substituiert ist,
R²¹ ein Halogenatom,
eine geradkettige oder verzweigte Alkylgruppe, die bis zu 3 Kohlenstoffatome enthält, welche gegebenenfalls mit einem oder mehreren Halogenatomen substituiert ist, oder
eine unter Nitro, Cyano, -S(O)ₙR⁷ und -OR⁷ ausgewählte Gruppe,
m 2 oder 3,
n 0, 1 oder 2,
q 0, 1 oder 2 und
t 1 oder 2.

3. Verbindung nach Anspruch 1, wobei bedeuten:
R eine geradkettige oder verzweigte Alkylgruppe, die bis zu 3 Kohlenstoffatome enthält, oder
eine Cycloalkylgruppe, die 3 oder 4 Kohlenstoffatome enthält, welche gegebenenfalls mit einer oder mehreren Methylgruppen substituiert ist,
R^{2,} R³ und R⁴, die gleich oder voneinander verschieden sein können, jeweils ein Wasserstoff-, Chlor-, Brom- oder Fluoratom oder
eine geradkettige oder verzweigte Alkyl- oder Alkenylgruppe, die bis zu 4 Kohlenstoffatome enthält, welche gegebenenfalls mit einem oder mehreren Chlor-, Brom- oder Fluoratomen substituiert ist,
eine geradkettige oder verzweigte Alkinylgruppe, die bis zu 4 Kohlenstoffatome enthält,
eine geradkettige oder verzweigte Alkylgruppe, die bis zu 4 Kohlenstoffatome enthält, welche durch -OR⁶ substituiert ist, oder
eine Gruppe, die unter -COR⁷, -CO₂R⁶, -S(O)ₙR⁹, -O(CH₂)ₘOR⁶, -N(R¹²)SO₂R⁸, -OR⁶¹, -(CR¹³R¹⁴)ₜS(O)_{q}R⁸ und -OSO₂R⁸ ausgewählt ist,
mit der Maßgabe, daß mindestens eine der Gruppen R² bis R⁴ -(CR¹³R¹⁴)ₜ-S(O)_{q}R⁸ darstellt,
R⁵ das Wasserstoffatom,
R⁶, R⁷, R⁸ und R⁹, die gleich oder voneinander verschieden sein können, jeweils
eine geradkettige oder verzweigte Alkylgruppe, die bis zu 4 Kohlenstoffatome enthält, welche gegebenenfalls mit einem oder mehreren Chlor-, Brom- oder Fluoratomen substituiert ist, oder
eine Cyclopropylgruppe,
R⁶¹ eine geradkettige oder verzweigte Alkyl- oder Alkenylgruppe, die bis zu 4 Kohlenstoffatome enthält, welche gegebenenfalls mit einem oder mehreren Chlor-, Brom- oder Fluoratomen substituiert ist,
eine geradkettige oder verzweigte Alkinylgruppe, die 3 oder 4 Kohlenstoffatome enthält, oder
eine Cyclopropylgruppe,
R¹² das Wasserstoffatom oder
eine geradkettige oder verzweigte Alkyl- oder Alkenylgruppe, die bis zu 4 Kohlenstoffatom enthält, welche gegebenenfalls mit einem oder mehreren Chlor-, Brom- oder Fluoratomen substituiert ist,
eine geradkettige oder verzweigte Alkinylgruppe, die 3 oder 4 Kohlenstoffatome enthält, oder
eine Cyclopropylgruppe,
R¹³ und R¹⁴, die gleich oder voneinander verschieden sein können, jeweils
das Wasserstoffatom oder
eine geradkettige oder verzweigte Alkylgruppe, die bis zu 3 Kohlenstoffatom enthält,
m 2 oder 3,
n 0, 1 oder 2,
q 0, 1 oder 2 und
t 1.

4. Verbindung nach Anspruch 1, wobei bedeuten:
R eine Methyl-, Ethyl-, Isopropyl-, Cyclopropyl- oder 1-Methylcyclopropylgruppe,
R^{2,} R³ und R⁴, die gleich oder voneinander verschieden sein können, jeweils
ein Wasserstoff-, Chlor-, Brom- oder Fluoratom oder
eine geradkettige oder verzweigte Alkyl- oder Alkenylgruppe, die bis zu 4 Kohlenstoffatom enthält, welche gegebenenfalls mit einem oder mehreren Chlor-, Brom- oder Fluoratomen substituiert ist,
eine geradkettige oder verzweigte Alkylgruppe, die bis zu 3 Kohlenstoffatome enthält, die durch -OR⁶ substituiert ist, oder
eine Gruppe, die unter -COR⁷, -CO₂R⁶, -SR⁹, -O(CH₂)ₘOR⁶, -OR⁶¹, -N(R¹²)SO₂R⁸, -OSO₂R⁸ und -(CR¹³R¹⁴)ₜS(O)_{q}R⁸ ausgewählt ist,
mit der Maßgabe, daß mindestens eine der Gruppen R² bis R⁴ -(CR¹³R¹⁴)ₜ-S(O)_{q}R⁸ darstellt,
R⁵ das Wasserstoffatom,
R⁶, R⁷, R⁸ und R⁹, die gleich oder voneinander verschieden sein können, jeweils
eine geradkettige oder verzweigte Alkylgruppe, die bis zu 3 Kohlenstoffatome enthält,
R⁶¹ eine geradkettige oder verzweigte Alkylgruppe, die bis zu 4 Kohlenstoffatome enthält, welche gegebenenfalls mit einem oder mehreren Chlor-, Brom- oder Fluoratomen substituiert ist,
eine geradkettige oder verzweigte Alkenyl- oder Alkinylgruppe, die 3 bis 4 Kohlenstoffatome enthält, oder
eine Cyclopropylgruppe,
R¹² das Wasserstoffatom oder
eine geradkettige oder verzweigte Alkylgruppe, die bis zu 3 Kohlenstoffatome enthält, welche gegebenenfalls mit einem oder mehreren Chlor-, Brom- oder Fluoratomen substituiert ist,
eine Allylgruppe, die gegebenenfalls mit einem oder mehreren Chlor-, Brom- oder Fluoratomen substituiert ist,
R¹³ und R¹⁴, die gleich oder voneinander verschieden sein können, jeweils das Wasserstoffatom oder eine Methyl- oder Ethylgruppe,
m 2 oder 3,
q 0, 1 oder 2 und
t 1.

5. Verbindung nach Anspruch 1, wobei bedeuten:
(a) R Cyclopropyl, R¹ Wasserstoff, R² -CH₂SO₂CH₃, R³ Wasserstoff, R⁴ Brom und R⁵ Wasserstoff,
(b) R Cyclopropyl, R¹ Wasserstoff, R² Chlor, R³ Wasserstoff, R⁴ -CH₂SCH₃ und R⁵ Wasserstoff,
(c) R Cyclopropyl, R¹ Wasserstoff, R² -CH₂SCH₃, R³ Brom, R⁴ Chlor und R⁵ Wasserstoff,
(d) R Cyclopropyl, R¹ Wasserstoff, R² -CH₂SO₂CH₂CH₃, R³ und R⁴ Chlor und R⁵ Wasserstoff,
(e) R Cyclopropyl, R¹ Wasserstoff, R² -CH₂SCH₃, R³ Brom und R⁴ und R⁵ Wasserstoff,
(f) R Cyclopropyl, R¹ Wasserstoff, R² -CH₂SO₂CH₃ und R³, R⁴ und R⁵ Wasserstoff,
(g) R Cyclopropyl, R¹ Wasserstoff, R² -CH₂SCH₃, R³ und R⁴ Fluor und R⁵ Wasserstoff,
(h) R Cyclopropyl, R¹ Wasserstoff, R² Chlor, R³ Wasserstoff, R⁴ -CH₂SCH₃ und R⁵ Wasserstoff,
(i) R Cyclopropyl, R¹ Wasserstoff, R² -SCH₃, R³ -CH₂SCH(CH₃)CH₃, R⁴ Chlor und R⁵ Wasserstoff,
oder ein landwirtschaftlich akzeptables Salz, ein Metallkomplex oder eine enoltautomere Form davon.

6. Herbicide Zusammensetzung, die eine herbicid wirksame Menge eines 2-Cyano-1,3-dion-Derivats der Formel (I) nach einem der Ansprüche 1 bis 5, oder ein landwirtschaftlich akzeptables Salz, Metallkomplex oder enoltauomere Form davon, in Verbindung mit einem landwirtschaftlich akzeptablen Verdünnungsmittel oder Träger und/oder grenzflächenaktiven Mittel enthält.

7. Herbicide Zusammensetzung nach Anspruch 6 in Form von wäßrigen Suspensionskonzentraten, benetzbaren Pulvern, wasserlöslichen oder in Wasser dispergierbaren Pulvern, flüssigen, wasserlöslichen Konzentraten, flüssigen, emulgierbaren Suspensionskonzentraten, Körnern oder emulgierbaren Konzentraten.

8. Verfahren zur Bekämpfung des Wachstums von Unkräutern an einer Örtlichkeit, bei dem auf die Örtlichkeit eine herbizid wirksame Menge eines in einem der Ansprüche 1 bis 5 definierten 2-Cyano-1,3-dion-Derivats der Formel (I) oder eines landwirtschaftlich akzeptablen Salzes, eines Metallkomplexes oder einer enoltautomeren Form davon, ausgebracht wird.

9. Verfahren nach Anspruch 8, wobei die Örtlichkeit eine Fläche ist, die zum Anbau von Kulturpflanzen verwendet wird oder verwendet werden soll, und die Verbindung in einer Ausbringmenge von 0,01 kg bis 4,0 kg/ha ausgebracht wird.

10. Verfahren zur Herstellung eines in Anspruch 1 definierten 2-Cyano-1,3-dion-Derivats der Formel (I), bei dem:
(a) eine Verbindung der Formel (II): 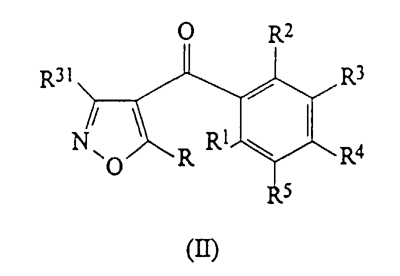 in der R, R¹, R^{2,} R³, R⁴ und R⁵ wie in Anspruch 1 definiert sind und R³¹ das Wasserstoffatom oder eine Acyl-Gruppe darstellt, mit einer Base umgesetzt wird, oder
(b) eine Verbindung der obigen Formel (II), wobei R³¹ einen Ester, ein Amid oder ein Nitril darstellt, der sauren oder basischen Hydrolyse unterzogen wird, oder
(c) eine Verbindung der Formel (III): 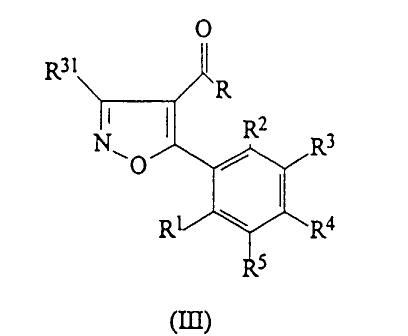 in der R, R¹, R², R³, R⁴ und R⁵ wie in Anspruch 1 definiert sind und R³¹ Wasserstoff oder eine Acylgruppe ist, mit einer Base umgesetzt wird, oder
(d) eine Verbindung der obigen Formel (III), wobei R³¹ einen Ester, ein Amid oder ein Nitril darstellt, der sauren oder basischen Hydrolyse unterzogen wird, oder
(e) ein Benzoylchlorid der Formel (IV): 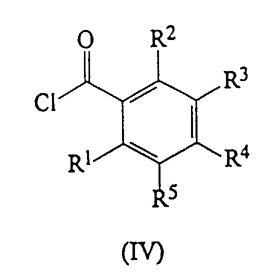 in der R¹, R², R³, R⁴ und R⁵ wie in Anspruch 1 definiert sind, mit einem β-Ketonitril der Formel (V): 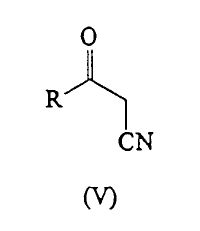 wobei R wie in Anspruch 1 definiert ist, umgesetzt wird, oder
(f) ein Säurechlorid der Formel (VI): 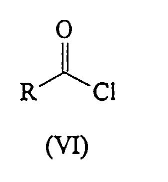 wobei R wie in Anspruch 1 definiert ist, mit einem β-Ketonitril der Formel (VII): 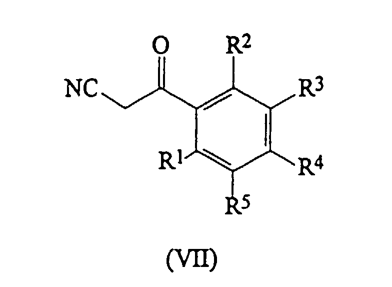 in der R¹, R², R³, R⁴ und R⁵ wie in Anspruch 1 definiert sind, umgesetzt wird, oder
(g) ein Benzoylchlorid der obigen Formel (IV) mit einem β-Ketonitril der Formel (V) in Gegenwart einer milden Base unter Bildung eines Zwischenprodukts der Formel (VIII): 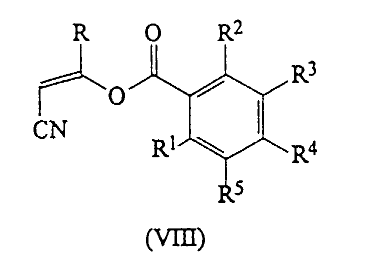 in der R, R¹, R^{2,} R³, R⁴ und R⁵ wie in Anspruch 1 definiert sind, umgesetzt wird, worauf das Zwischenprodukt der Formel (VIII) in Gegenwart eines Katalysators umgelagert wird, oder
(h) ein Säurechlorid der oben definierten Formel (VI) mit einem β-Ketonitril der oben definierten Formel (VII) in Gegenwart einer milden Base unter Bildung eines Zwischenprodukts der Formel (IX): 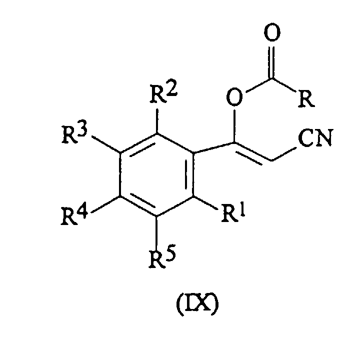 in der R, R¹, R², R³, R⁴ und R⁵ wie in Anspruch 1 definiert sind, umgesetzt wird, worauf das Zwischenprodukt der Formel (IX) in Gegenwart eines Katalysators umgelagert wird,
worauf sich gegebenenfalls die Umwandlung der so erhaltenen Verbindung in ein landwirtschaftlich akzeptables Salz oder Metallkomplex davon anschließt.

## Revendications

1. Nouveaux dérivés de la 2-cyano-1,3-dione de formule générale (I) 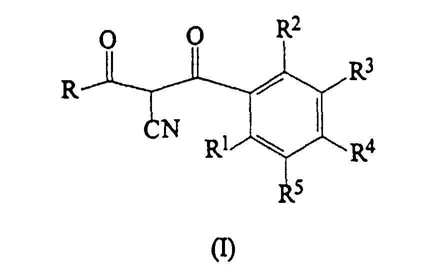 dans laquelle :
R représente:,
un radical alkyle, linéaire ou ramifié, contenant de 1 à 6 atomes de carbone, éventuellement substitué par un ou plusieurs atomes d'halogènes ; ou
un radical cycloalkyle, contenant de 3 à 6 atomes de carbone, éventuellement substitué par un ou plusieurs radicaux R⁷ ;
R¹ représente un atome d'hydrogène ;
R², R³, R⁴ et R⁵, qui peuvent être identiques ou différents, représentent chacun :-
un atome d'hydrogène ;
un atome d'halogène ;
un radical alkyle, alkényle ou alkynyle linéaire ou ramifié, contenant de 1 à 6 atomes de carbone, éventuellement substitué par un ou plusieurs atomes d'halogènes ; ou
Un radical phényle éventuellement substitué par de 1 à 3 radicaux R²¹ qui peuvent être identiques ou différents ;
un radical alkyle, linéaire ou ramifié, contenant de 1 à 6 atomes de carbone, éventuellement substitué par un radical OR⁶ ;
un radical choisi parmi les radicaux suivants ; nitro, cyano, -CO₂R⁶, -COR⁷, -S(O)ₙR⁹, -O(CH₂)ₘOR⁶, N(R¹²)SO₂R⁸, OR⁶¹, -OSO₂R⁸, CONR¹⁰R¹⁵ et (CR¹³R¹⁴)ₜ-S(O)_{q}R⁸ ;
à condition que au moins un des radicaux R² à R⁵ représente -(CR¹³R¹⁴)ₜ-S(O)_{q}R⁸ ;
R⁶ représente :
un radical alkyle, alkényle ou alkynyle linéaire ou ramifié, contenant de 1 à 6 atomes de carbone, éventuellement substitué par un ou plusieurs atomes d'halogènes ; ou
un radical cycloalkyle, contenant de 3 à 6 atomes de carbone ;
R⁶¹ représente :
un radical alkyle, alkényle ou alkynyle linéaire ou ramifié, contenant de 1 à 6 atomes de carbone, éventuellement substitué par un ou plusieurs atomes d'halogènes ; ou
un radical cycloalkyle, contenant de 3 à 6 atomes de carbone ;
un radical phényle éventuellement substitué par de 1 à 3 radicaux R²¹ qui peuvent être identiques ou différents ;
R⁷ représente
un radical alkyle, linéaire ou ramifié, contenant de 1 à 6 atomes de carbone, éventuellement substitué par un ou plusieurs atomes d'halogènes ; ou
un radical cycloalkyle, contenant de 3 à 6 atomes de carbone ;
R⁸ représente :
un radical alkyle, alkényle ou alkynyle linéaire ou ramifié, contenant de 1 à 6 atomes de carbone, éventuellement substitué par un ou plusieurs atomes d'halogènes ; ou
un radical cycloalkyle, contenant de 3 à 6 atomes de carbone ;
un radical phényle éventuellement substitué par de 1 à 5 radicaux R²¹ qui peuvent être identiques ou différents ; ou
-NR¹⁰R¹¹ ;
R⁹ représente :
un radical alkyle, alkényle ou alkynyle linéaire ou ramifié, contenant de 1 à 6 atomes de carbone, éventuellement substitué par un ou plusieurs atomes d'halogènes ; ou
un radical cycloalkyle, contenant de 3 à 6 atomes de carbone ;
un radical phényle éventuellement substitué par de 1 à 3 radicaux R²¹ qui peuvent être identiques ou différents ;
R¹⁰ représente un atome d'hydrogène ou un radical alkyle, linéaire ou ramifié, contenant de 1 à 6 atomes de carbone, éventuellement substitué par un ou plusieurs atomes d'halogènes ;
R¹¹ représente :
un radical alkyle, linéaire ou ramifié, contenant de 1 à 6 atomes de carbone, éventuellement substitué par un ou plusieurs atomes d'halogènes ; ou
un radical choisi parmi les radicaux suivants : -COR⁷, -CO₂R⁷ et CONR⁷R¹⁰ ;
lorsque R¹⁰ et R¹¹ font partie d'un radical -NR¹⁰R¹¹ ils peuvent former, avec l'atome d'azote auquel ils sont attachés, un cycle à 5 ou 6 chaînons, contenant éventuellement dans le cycle un autre hétéroatome tel que l'oxygène ou l'azote, le dit-cycle étant éventuellement substitué par un ou plusieurs radicaux alkyle contenant de 1 à 3 atomes de carbone.
R¹² représente :
un atome d'hydrogène ;
un radical alkyle, alkényle ou alkynyle linéaire ou ramifié, contenant de 1 à 6 atomes de carbone, éventuellement substitué par un ou plusieurs atomes d'halogènes ; ou
un radical cycloalkyle, contenant de 3 à 6 atomes de carbone ;
Un radical phényle éventuellement substitué par de 1 à 5 radicaux R²¹ qui peuvent être identiques ou différents ;
ou un radical -OR¹⁷ ;
R¹³ et R¹⁴, qui peuvent être identiques ou différents, représentent chacun un atome d'hydrogène ou un radical alkyle, linéaire ou ramifié, contenant de 1 à 6 atomes de carbone, éventuellement substitué par un ou plusieurs atomes d'halogènes
R¹⁵ représente un radical choisi parmi les radicaux R⁷ et -OR¹⁷ ;
lorsque R¹⁰ et R¹⁵ font partie d'un radical -CONR¹⁰R¹⁵ ils peuvent former, avec l'atome d'azote auquel ils sont attachés, un cycle à 5 ou 6 chaînons, contenant éventuellement dans le cycle un autre hétéroatome tel que l'oxygène ou l'azote, le dit-cycle étant éventuellement substitué par un ou plusieurs radicaux alkyle contenant de 1 à 3 atomes de carbone.
^{R17} représente un radical alkyle, linéaire ou ramifié, contenant de 1 à 6 atomes de carbone ;
R²¹ représente :
un atome d'halogène ;
un radical alkyle, linéaire ou ramifié, contenant de 1 à 3 atomes de carbone, éventuellement substitué par un ou plusieurs atomes d'halogènes ; ou
un radical choisi parmi les radicaux suivants ; nitro, cyano, -S(O)ₙR⁷ et -OR⁷ ;
m représente 0, 2 ou 3
n représente 0, 1 ou 2;
q représente 0, 1 ou 2 ; et
t représente un un nombre entier pouvant prendre toutes les valeurs de 1 à 4 ; si t est supérieur à 1 les radicaux -(CR¹³R¹⁴) peuvent être identiques ou différents ;

2. Un composé selon la revendication 1 dans laquelle :
R représente:
un radical alkyle, linéaire ou ramifié, contenant de 1 à 6 atomes de carbone, éventuellement substitué par un ou plusieurs atomes d'halogènes
un radical cycloalkyle, contenant de 3 à 6 atomes de carbone, éventuellement substitué par un ou plusieurs radicaux méthyle ;
R², R³, R⁴ et R⁵ qui peuvent être identiques ou différents représentent chacun :
un atome d'hydrogène ou un atome d'halogène ;
un radical alkyle, alkényle ou alkynyle linéaire ou ramifié, contenant de 1 à 6 atomes de carbone, éventuellement substitué par un ou plusieurs atomes d'halogènes;
un radical alkyle, linéaire ou ramifié, contenant de 1 à 6 atomes de carbone, éventuellement substitué par un radical OR⁶ ;
un radical choisi parmi les radicaux suivants ; -COR⁷, -CO₂R⁶, nitro, cyano, -O(CH₂)ₘOR⁶, OR⁶¹, N(R¹²)SO²R⁸ ⁻OSO₂R⁸, S(O)ₙR⁹, et -(CR¹³R¹⁴)ₜ-S(O)_{q}R⁸ ;
R⁶ et R⁷ qui peuvent être identiques ou différents représentent chacun :
un radical alkyle, linéaire ou ramifié, contenant de 1 à 6 atomes de carbone, éventuellement substitué par un ou plusieurs atomes d'halogènes ; ou
un radical cycloalkyle, contenant de 3 ou 4 atomes de carbone ;
R⁶¹ et R⁹ qui peuvent être identiques ou différents représentent chacun :
un radical alkyle ou alkényle linéaire ou ramifié, contenant de 1 à 6 atomes de carbone, éventuellement substitué par un ou plusieurs atomes d'halogènes ; ou
un radical cycloalkyle, contenant de 3 à 6 atomes de carbone ;
R⁸ représente :
un radical alkyle ou alkényle linéaire ou ramifié, contenant de 1 à 6 atomes de carbone, éventuellement substitué par un ou plusieurs atomes d'halogènes ; ou
un radical alkynyle linéaire ou ramifié, contenant de 3 à 6 atomes de carbone, éventuellement substitué par un ou plusieurs atomes d'halogènes ; ou
un radical cycloalkyle, contenant de 3 à 6 atomes de carbone ;
un radical phényle éventuellement substitué par de 1 à 3 radicaux R²¹ qui peuvent être identiques ou différents ;.
R¹² représente :
un atome d'hydrogène ;
un radical alkyle ou alkényle linéaire ou ramifié, contenant de 1 à 6 atomes de carbone, éventuellement substitué par un ou plusieurs atomes d'halogènes ;
un radical alkynyle linéaire ou ramifié, contenant de 3 à 6 atomes de carbone, éventuellement substitué par un ou plusieurs atomes d'halogènes ; ou
un radical cycloalkyle, contenant de 3 à 6 atomes de carbone ;
R¹³ et R¹⁴, qui peuvent être identiques ou différents, représentent chacun ;
un atome d'hydrogène ou un radical alkyle, linéaire ou ramifié, contenant de 1 à 6 atomes de carbone, éventuellement substitué par un ou plusieurs atomes d'halogènes
R²¹ représente :
un atome d'halogène ;
un radical alkyle, linéaire ou ramifié, contenant de 1 à 3 atomes de carbone, éventuellement substitué par un ou plusieurs atomes d'halogènes ; ou
un radical choisi parmi les radicaux suivants ; nitro, cyano, -S(O)ₙR⁷ et -OR⁷ ;
m représente 2 ou 3
n représente 0, 1 ou 2;
q représente 0, 1 ou 2 ; et
t représente 1 ou 2

3. Un composé selon la revendication 1 dans laquelle :
R représente :
un radical alkyle, linéaire ou ramifié, contenant de 1 à 3 atomes de carbone ;
un radical cycloalkyle, contenant de 3 à 4 atomes de carbone, éventuellement substitué par un ou plusieurs radicaux méthyle ;
R^{2,} R³, et R⁴ qui peuvent être identiques ou différents représentent chacun :
un atome d'hydrogène ou un atome de chlore, de brome ou de fluor ; ou
un radical alkyle,alkényle ou linéaire ou ramifié, contenant de 1 à 4 atomes de carbone, éventuellement substitué par un ou plusieurs atomes de chlore, de brome ou de fluor ; ou
un radical alkynyle linéaire ou ramifié, contenant de 1 à 4 atomes de carbone ;
un radical alkyle, linéaire ou ramifié, contenant de 1 à 4 atomes de carbone substitué par un radical OR⁶ ;
un radical choisi parmi les radicaux suivants ; -COR⁷, -CO₂R⁶, -S(O)ₙR⁹, -O(CH₂)ₘOR⁶, -N(R¹²)SO²R⁸ , -OR⁶¹,- (CR¹³R¹⁴)ₜ-S(O)_{q}R⁸ ; et OSO₂R⁸,
à condition que au moins l'un des radicaux R² à R⁴ représente un radical (CR¹³R¹⁴)ₜ-S(O)_{q}R⁸ ;
R⁵ représente un atome d'hydrogène
R⁶, R⁷, R⁸ et R⁹ qui peuvent être identiques ou différents représentent chacun :
un radical alkyle, linéaire ou ramifié, contenant de 1 à 4 atomes de carbone, éventuellement substitué par un ou plusieurs atomes de chlore, de brome ou de fluor ; ou
un radical cyclopropyle ;
R⁶¹ représente :
un radical alkyle ou alkényle linéaire ou ramifié, contenant de 1 à 4 atomes de carbone, éventuellement substitué par un ou plusieurs atomes de chlore, de brome ou de fluor ; ou
un radical alkynyle linéaire ou ramifié, contenant de 3 à 4 atomes de carbone ; ou
un radical cyclopropyle ;
R¹² représente :
un atome d'hydrogène ; ou
un radical alkyle ou alkényle linéaire ou ramifié, contenant de 1 à 4 atomes de carbone, éventuellement substitué par un ou plusieurs atomes de chlore, de brome ou de fluor ; ou
un radical alkynyle linéaire ou ramifié, contenant de 3 à 4 atomes de carbone ;
un radical cyclopropyle ;
R¹³ et R¹⁴, qui peuvent être identiques ou différents, représentent chacun :
un atome d'hydrogène ou ;
un radical alkyle, linéaire ou ramifié, contenant de 1 à 3 atomes de carbone ;
m représente 2 ou 3
n représente 0, 1 ou 2 ;
q représente 0, 1 ou 2 ; et
t représente 1

4. Un composé selon la revendication 1; dans laquelle celle dans laquelle :
R représente :
un radical méthyle, éthyle, isopropyle, cyclopropyle ou 1 -méthylcyclopropyle
R², R³ et R⁴ qui peuvent être identiques ou différents représentent chacun :
un atome d'hydrogène ou un atome de chlore, de brome ou de fluor ; ou
un radical alkyle,alkényle ou linéaire ou ramifié, contenant de 1 à 4 atomes de carbone, éventuellement substitué par un ou plusieurs atomes de chlore, de brome ou de fluor ;
un radical alkyle, linéaire ou ramifié, contenant de 1 à 3 atomes de carbone substitué par un radical OR⁶ ;
un radical choisi parmi les radicaux suivants ; -COR⁷, -CO₂R⁶, -SR⁹, -O(CH₂)ₘOR⁶, -OR⁶¹, -N(R¹²)SO²R⁸, OSO₂R⁸, -(CR¹³R¹⁴)ₜ-S(O)_{q}R⁸ ;
à condition que au moins l'un des radicaux R² à R⁴ représente un radical (CR¹³R¹⁴)ₜ-S(O)_{q}R⁸ ;
R⁵ représente un atome d'hydrogène
R⁶, R⁷, R⁸ et R⁹ qui peuvent être identiques ou différents représentent chacun:
un radical alkyle, linéaire ou ramifié, contenant de 1 à 3 atomes de carbone ;
R⁶¹ représente :
un radical alkyle linéaire ou ramifié, contenant de 1 à 4 atomes de carbone, éventuellement substitué par un ou plusieurs atomes de chlore, de brome ou de fluor ; ou
un radical alkyle ou alkényle linéaire ou ramifié, contenant de 3 à 4 atomes de carbone ; ou
un radical cyclopropyle ;
R¹² représente :
un atome d'hydrogène ; ou
un radical alkyle ou alkényle linéaire ou ramifié, contenant de 1 à 3 atomes de carbone, éventuellement substitué par un ou plusieurs atomes de chlore, de brome ou de fluor ; ou
un radical allyle éventuellement substitué par un ou plusieurs atomes de chlore, de brome ou de fluor ; ou
R¹³ et R¹⁴, qui peuvent être identiques ou différents, représentent chacun un atome d'hydrogène ou un radical méthyle ou éthyle ;
m représente 2 ou 3;
n représente 0, 1 ou 2 ;
q représente 0, 1 ou 2 ; et
t représente 1

5. Un composé selon la revendication 1 dans laquelle
(a) R représente un radical cyclopropyle, R¹ représente l'hydrogène, R² représente un radical -CH₂SO₂CH₃, R³ représente l'hydrogène, R⁴ représente le brome et R⁵ représente l'hydrogène ;
(b) R représente un radical cyclopropyle, R¹ représente l'hydrogène, R² représente le chlore, R³ représente l'hydrogène, R⁴ représente -CH₂SCH₃ et R⁵ représente l'hydrogène ;
(c) R représente un radical cyclopropyle, R¹ représente l'hydrogène, R² représente un radical -CH₂SCH₃, R³ représente le brome, R⁴ représente le chlore et R⁵ représente l'hydrogène ;
(d) R représente un radical cyclopropyle, R¹ représente l'hydrogène, R² représente un radical -CH₂SO₂ CH₂CH₃, R³ et R⁴ représentent le chlore et R⁵ représente l'hydrogène ;
(e) R représente un radical cyclopropyle, R¹ représente l'hydrogène, R² représente un radical -CH₂SCH₃, R³ représente le brome, R⁴ et R⁵ représentent l'hydrogène ;
(f) R représente un radical cyclopropyle, R¹ représente l'hydrogène, R² représente un radical -CH₂SO₂CH₃, R³, R⁴ et R⁵ représentent l'hydrogène ;
(g) R représente un radical cyclopropyle, R' représente l'hydrogène, R² représente un radical -CH₂SCH₃, R³ et R⁴ représentent le fluor et R⁵ représente l'hydrogène ;
(h) R représente un radical cyclopropyle, R' représente l'hydrogène, R² représente le chlore, R³ représente l'hydrogène, R⁴ représente -CH₂SCH₃ et R⁵ représente l'hydrogène ;
(i) R représente un radical cyclopropyle, R¹ représente l'hydrogène, R² représente un radical SCH₃, R₃ représente un radical -CH₂SCH(CH₃)CH₂, R⁴ représente le chlore et R⁵ représente l'hydrogène ;
ou un sel ou un complexe métallique énolique ou tautomère de ces composés acceptable pour des usages agricoles.

6. Une composition herbicide contenant comme matière active une quantité herbicide efficace d'un dérivé de la 2-cyano-1,3-dione de formule générale (I), selon l'une quelconque des revendications 1 à 5 ou un sel, ou un complexe métallique énolique ou tautomère de ces composés acceptable pour des usages agricoles, associé avec des diluants ou des charges et/ou des agents tensio-actifs acceptables pour des applicationsagricoles.

7. Une composition herbicide selon la revendication 6 sous forme d'une suspension aqueuse concentrée, d'une poudre mouillable, d'une poudre dispersable ou soluble dans l'eau, d'un concentré liquide soluble dans l'eau, d'une suspension liquide concentrée émulsionnable, d'un granulé ou d'un concentré émulsionnable.

8. Un procédé de contrôle de la croissance des mauvaises herbes dans un lieu consistant à appliquer sur le dit-lieu une quantité herbicide efficace d'un dérivé de la 2-cyano-1,3-dione de formule générale (I) comme défini dans l'une quelconque des revendications 1 à 5, ou d'un sel, ou d'un complexe métallique énolique ou tautomère de ces composés acceptable pour des usages agricoles.

9. Un procédé selon la revendication 8 dans lequel le lieu est une zone utilisée ou destinée à être utilisée pour y faire pousser des cultures, et où la dose d'emploi utilisée est comprise entre 0,01 et 4 kg de matière active par hectare.

10. Un procédé de préparation d'un dérivé de la 2-cyano-1,3-dione de formule générale (I) tel que défini dans la revendication 1 qui comprend les étapes suivantes :
(a) action d'un composé de formule générale (II) : 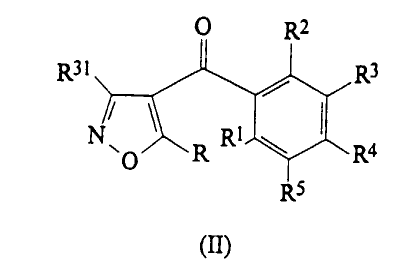 dans laquelle R, R¹, R², R³, R⁴ et R⁵ ont la définition donnée dans la revendication 1, et R³¹ représente un atome d'hydrogène ou un radical acyle, sur une base, ou
(b) hydrolyse basique ou acide d'un composé de formule générale (II) ci-dessus dans laquelle R³¹ représente un radical ester, amide ou nitrile ; ou
(c) action d'un composé de formule générale (III) 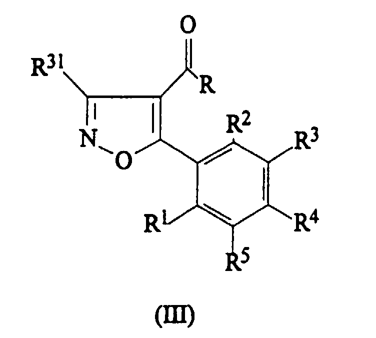 dans laquelle R, R¹, R², R³, R⁴ R⁵ et R³¹ ont la définition donnée dans la revendication 1 et R³¹ représente l'hydrogène ou un radical acyle, sur une base ; ou
(d) hydrolyse acide ou basique d'un composé de formule générale (III) ci-dessus dans laquelle R³¹ représente un radical ester, amide ou nitrile ;
(e) action d'un chlorure de benzoyle de formule générale (IV) : 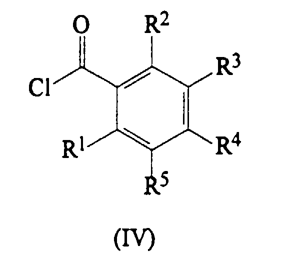 dans laquelle R, R¹, R², R³, R⁴ et R⁵ ont la définition donnée dans la revendication 1, sur un béta-cétonitrile de formule générale (V) : 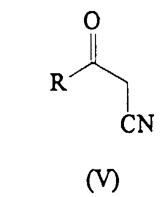 dans laquelle R a la définition donnée dans la revendication 1 ; ou
(f) action d'un chlorure dacide de formule générale (VI) : 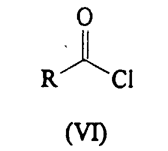 dans laquelle R a la définition donnée dans la revendication 1, sur un béta-cétonitrile de formule générale (VII) ; 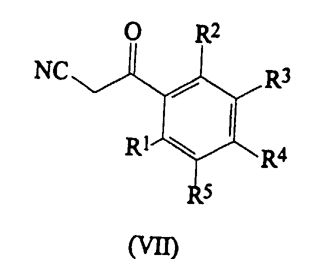 dans laquelle R, R¹, R², R³, R⁴ et R⁵ ont la définition donnée dans la revendication 1 ; ou
(g) action d'un chlorure de benzoyle de formule générale (IV) ci-dessus, sur un béta-cétonitrile de formule générale (V), en présence d'une base faible, pour former un intermédiaire de formule générale (VIII) 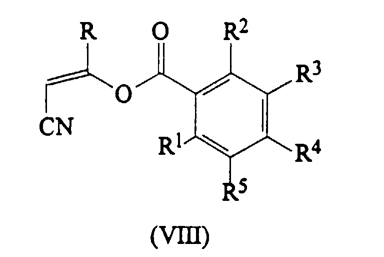 dans laquelle R¹, R², R³, R⁴ et R⁵ ont la définition donnée dans la revendication 1, suivie du réarrangement du composé intermédiaire de formule générale (VIII) en présence d'un catalyseur ; ou
(h) action d'un chlorure d'acide de formule générale (VI) ci-dessus, sur un béta-cétonitrile de formule générale (VII) ci-dessus, en présence d'une base faible, pour donner un intermédiaire de formule aénérale (IX) 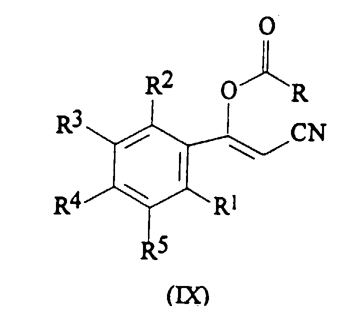 dans laquelle R¹, R², R³, R⁴ et R⁵ ont la définition donnée dans la revendication 1, suivi du réarrangement de l'intermédiaire de formule générale (IX) en présence d'un catalyseur ;
éventuellement suivi de la conversion du composé ainsi obtenu en un sel ou en un complexe métallique acceptable pour des usages agricoles, de ces composés.
